(19) Europäisches Patentamt
European Patent Office
Office européen des brevets

(11) Numéro de publication : **0 517 590 A1**

(12) # DEMANDE DE BREVET EUROPEEN

(21) Numéro de dépôt : **92401526.6**

(51) Int. Cl.[5] : **C07D 263/32,** A61K 31/42

(22) Date de dépôt : **04.06.92**

(30) Priorité : **06.06.91 FR 9106846**

(43) Date de publication de la demande : **09.12.92 Bulletin 92/50**

(84) Etats contractants désignés : **PT**

(71) Demandeur : **LABORATOIRE ROGER BELLON
159, avenue Achille Peretti
F-92201 Neuilly-sur-Seine (FR)**

(72) Inventeur : **Barreau, Michel
24 bis avenue de Clos Sénart
F-91230 Montgeron (FR)**
Inventeur : **Kryvenko, Michel
5 rue Franklin
F-75116 Paris (FR)**
Inventeur : **Lavergne, Marc Pierre
8 Sente de l'Espérance
F-94520 Mandres les roses (FR)**
Inventeur : **Techer, Auguste
2 route de Samois
F-77210 Avon (FR)**

(74) Mandataire : **Savina, Jacques et al
RHONE-POULENC RORER S.A., Direction des Brevets, 90 avenue Raymond Aron
F-92165 Antony Cédex (FR)**

(54) **Nouveaux dérivés de l'oxazole, leur préparation et les compositions qui les contiennent.**

(57) Nouveaux dérivés de l'oxazole de formule générale (I) dans laquelle R est un atome d'hydrogène ou un radical alcoyle (1 ou 2C), $R_1$ et $R_2$ sont identiques ou différents et représentent des atomes d'hydrogène ou d'halogène, ou des radicaux alcoyloxy dont la partie alcoyle contient 1 à 4 atomes de carbone en chaîne droite ou ramifiée, $R_3$, est $NH_2$ ou alcoyle droit ou ramifié (1 à 4C) et n égale 9 à 6, ainsi que leur sels, lorsqu'ils existent leurs isomères et leurs mélanges, et leur préparation.

Ces produits manifestent une activité anti-inflammatoire.

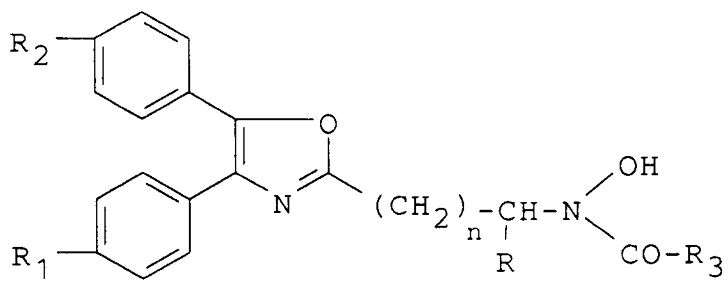

(I)

EP 0 517 590 A1

La présente invention concerne de nouveaux dérivés de l'oxazole de formule générale :

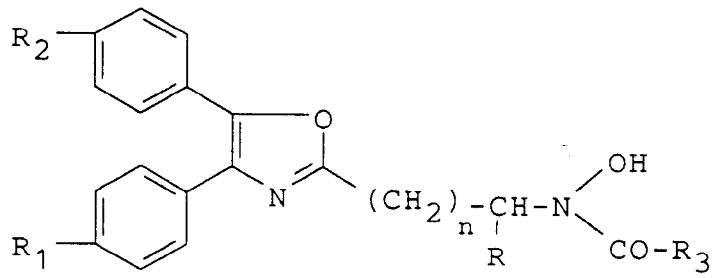

(I)

dans laquelle :

R est un atome d'hydrogène ou un radical alcoyle contenant 1 ou 2 atomes de carbone,

$R_1$ et $R_2$ sont identiques ou différents et représentent des atomes d'hydrogène ou d'halogène, ou des radicaux alcoyloxy dont la partie alcoyle contient 1 à 4 atomes de carbone en chaîne droite ou ramifiée,

$R_3$ représente un radical amino ou alcoyle contenant 1 à 4 atomes de carbone en chaine droite ou ramifiée et,

n égale 3 à 6,

ainsi que leurs sels, leurs isomères lorsqu'ils existent et les compositions pharmaceutiques qui les contiennent.

Dans la demande de brevet GB 1 206 403 ont été décrits les dérivés de l'oxazole de formule générale :

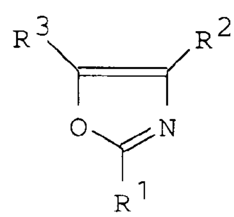

dans laquelle $R^2$ et $R^3$ sont identiques ou différents et représentent des radicaux aryle éventuellement substitués et $R^1$ est un radical acide aliphatique saturé ou insaturé contenant 2 à 6 atomes de carbone, ainsi que leurs sels, leurs esters et leurs amides ou les acides hydroxamiques correspondants.

Ces produits présentent une activité anti-inflammatoire.

Il a été trouvé maintenant, que les dérivés de l'oxazole selon l'invention manifestent une action antagoniste des effets du leucotriène $B_4$ et inhibitrice de l'action de la 5-lipoxygénase.

Le leucotriène $B_4$ est un puissant médiateur de l'inflammation qui est formé à la suite de la biotransformation de l'acide arachidonique par la voie de la 5-lipoxygénase. Il contribue en particulier à des phénomènes comme le chimiotactisme, l'activation cellulaire, l'exocytose d'enzymes et participe également aux dérèglements immunologiques et tissulaires. Dans la mesure où les anti-inflammatoires ne possèdent pas nécessairement cette activité, les produits selon l'invention sont particulièrement intéressants dans le traitement des maladies où ce médiateur joue un rôle.

Dans la formule générale (I), lorsque les radicaux $R_1$ et/ou $R_2$ représentent des atomes d'halogène, ils peuvent être choisis parmi les atomes de fluor, de chlore ou de brome. De préférence ils représentent l'atome de chlore. Le symbole n est compris entre 3 et 6, mais de préférence on choisira n égal à 4 ou 5.

Selon l'invention les nouveaux dérivés de l'oxazole peuvent être préparés à partir de l'hydroxylamine de formule générale :

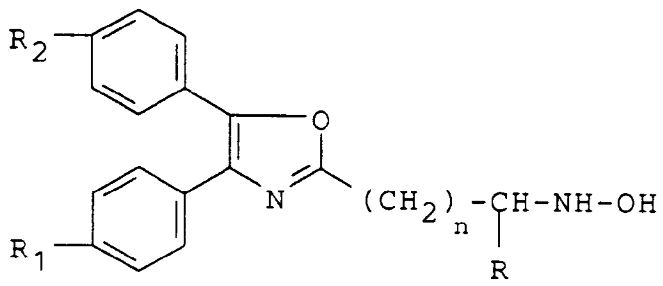

(II)

dans laquelle R, $R_1$, $R_2$ et n sont définis comme précédemment, ou de son sel, soit par action d'un cyanate alcalin, soit par acylation au moyen d'un dérivé réactif d'un acide de formule générale :

$R_3$-COOH (III)

dans laquelle $R_3$ est défini comme précédemment.

Le traitement par un cyanate alcalin conduit aux dérivés de l'oxazole de formule générale (I) pour lesquels $R_3$ est un radical amino. On opère de préférence au moyen de cyanate de potassium en milieu hydro-organique, par exemple dans un mélange eau-tétrahydrofuranne, à une température comprise entre 20°C et la température de reflux du mélange réactionnel.

L'acylation conduit aux dérivés de l'oxazole pour lesquels $R_3$ est un radical alcoyle, elle peut être effectuée par toute méthode connue qui n'affecte pas le reste de la molécule, notamment par le chlorure d'acide correspondant, en présence d'une base [(base alcaline diluée, carbonate (par exemple carbonate de potassium) ou base organique azotée (base organique tertiaire comme par exemple la triéthylamine), à une température comprise entre 0 et 150°C, dans un solvant organique chloré (chlorure de méthylène par exemple) ou un éther (tétrahydrofuranne ou dioxanne par exemple), dans un ester (acétate d'éthyle ou d'isopropyle) ou dans une cétone (acétone, butanone par exemple). Il est également possible d'opérer au moyen de l'anhydride en solution dans l'acide correspondant, à une température comprise entre 5 et 150°C ou éventuellement en présence d'un solvant chloré, d'un éther (dioxanne), de pyridine, ou en milieu aqueux en présence d'une base minérale puis de libérer le radical hydroxy par traitement par une base en milieu hydro-alcoolique. On opère avantageusement en présence d'ammoniaque en milieu hydro-méthanolique à une température voisine de 20°C.

Les dérivés de l'hydroxylamine de formule générale (II) pour lesquels R est un atome d'hydrogène, peuvent être obtenus par alcoylation du sel de sodium du méthyl-3 hydroxy-5 isoxazolecarboxylate-4 d'alcoyle suivie d'une hydrolyse acide selon le procédé de G. DOLESCHALL, Tet. Lett., 28, 2993 (1987), à partir d'un dérivé de l'oxazole de formule générale :

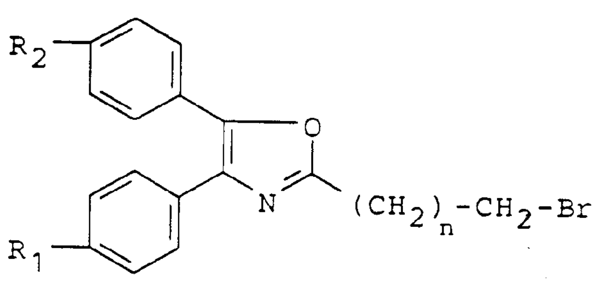

(III)

dans laquelle $R_1$, $R_2$ et n sont définis comme précédemment.

La condensation du dérivé isoxazolecarboxylate-4 sur le produit de formule générale (III) s'effectue dans un solvant organique tel qu'un amide (diméthylformamide par exemple), le diméthylsulfoxyde, le diméthylacétamide ou la N-méthylpyrrolidone en présence d'iodure de potassium par exemple à une température comprise entre 50 et 150°C.

L'hydrolyse acide du produit ainsi obtenu, de formule générale :

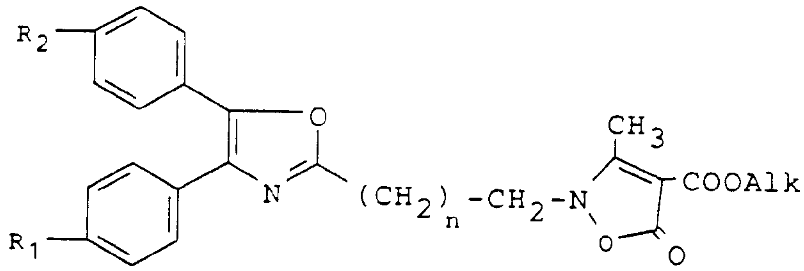

(IV)

dans laquelle $R_1$, $R_2$, et n sont définis comme précedemment et Alk représente un radical alcoyle droit ou ramifié contenant 1 à 4 atomes de carbone, s'effectue avantageusement en solution dans un mélange acide acétique/acide chlorhydrique concentré, à une température comprise entre 90°C et la température de reflux du mélange réactionnel.

Le dérivé bromé de formule générale (III) peut être obtenu par cyclisation du cétoamide de formule générale :

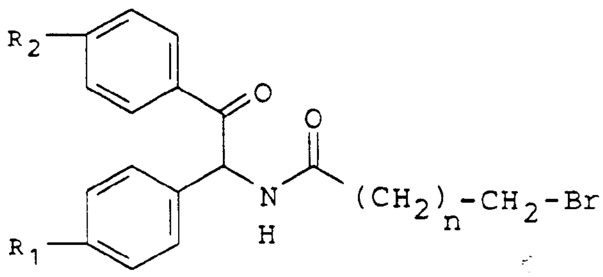

(V)

dans laquelle $R_1$, $R_2$ et n sont définis comme précédemment.

On opère en présence d'un agent de déshydratation comme par exemple l'oxychlorure de phosphore, le chlorure de thionyle, le tribromure de phosphore,l'acide chlorosulfonique ou en présence d'un arylsulfochlorure (chlorure de benzènesulfonyle, chlorure de tosyle) dans la pyridine. La réaction s'effectue avec ou sans solvant à une température comprise entre 5 et 150°C. Lorsque l'on opère dans un solvant, celui-ci est avantageusement choisi parmi le cyclohexane, les solvants aromatiques (toluène) ou les solvants chlorés (chlorure de méthylène, dichloro-1,2 éthane).

Le dérivé bromé de formule générale (V) peut être obtenu par action du chlorure de l'acide de formule générale :

$$Cl\text{-}CO\text{-}(CH_2)_n\text{-}CH_2\text{-}Br \qquad (VI)$$

dans laquelle n est défini comme précédemment, sur le chlorhydrate de l'aminocétone de formule générale :

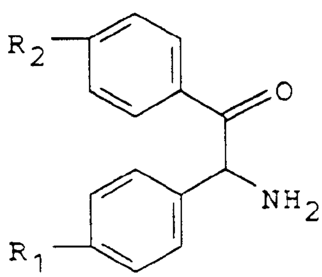

(VII)

dans laquelle $R_1$ et $R_2$ sont définis comme précédemment.

La réaction s'effectue généralement en présence d'un excès du chlorure d'acide de formule générale (VI), et d'une base organique azotée comme la pyridine ou une amine tertiaire (triéthylamine, N-méthylmorpholine ou N,N-diméthylaniline par exemple), ou d'un carbonate alcalin. On opère avantageusement dans un solvant chloré (chloroforme, chlorure de méthylène), dans un éther (éther éthylique, tétrahydrofuranne, dioxanne, diméthoxy-1,2 éthane) ou dans un hydrocarbure aliphatique ou aromatique, à une température comprise entre 5 et 120°C.

Le chlorhydrate de l'aminocétone de formule générale (VII) peut être préparé selon les méthodes décrites

par :

- G. Drefahl et Coll., Ann. Chem., 589, 82 (1954) et J. Prakt. Chem., 32, 307 (1966);
- M.J. Hatch et Coll., J. Am. Chem. Soc., 75, 38 (1953);
- H.O. House et Coll., J. Org. Chem., 28, 307 (1963); ou comme décrit ci-après dans les exemples.

Le dérivé de l'hydroxylamine de formule générale (II) peut également être obtenu par réduction de l'oxime de formule générale:

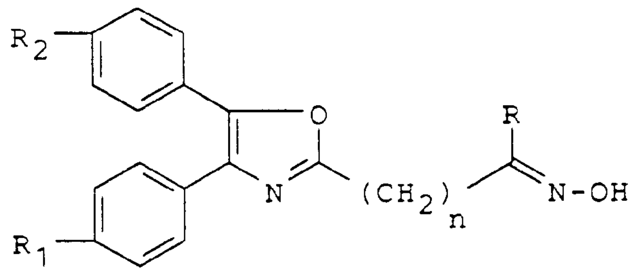

(VIII)

dans laquelle $R_1$, $R_2$, R, et n sont définis comme précédemment.

La réaction s'effectue avantageusement en milieu acétique au moyen d'un agent réducteur comme par exemple un cyanoborohydrure alcalin, à une temperature comprise entre 5 et 50°C selon la méthode décrite par G. W. Gribble et coll., Synthesis, 856 (1977).

Il est également possible d'effectuer la réduction selon les méthodes décrites par I.D.M. Herscheid et coll., Tet. Lett., 51, 5143 (1978) ou par M. W. Tijhuis et coll., Synthesis 890 (1980).

L'oxime de formule générale (VII) peut être préparé par action de l'hydroxylamine sur la cétone correspondante de formule générale :

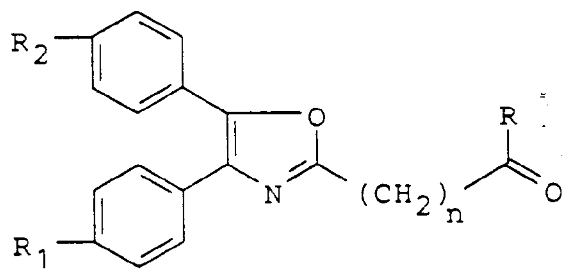

(IX)

dans laquelle $R_1$, $R_2$, R et n sont définis comme précédemment.

La réaction s'effectue généralement par action du chlorhydrate d'hydroxylamine, en présence d'une base comme par exemple la soude, un carbonate ou une base azotée (pyridine), en milieu hydroalcoolique (éthanol aqueux), à une température comprise entre 20 et 80°C.

La cétone de formule générale (IX) pour laquelle R est un atome d'hydrogène, peut être obtenue par oxydation de l'alcool correspondant de formule générale :

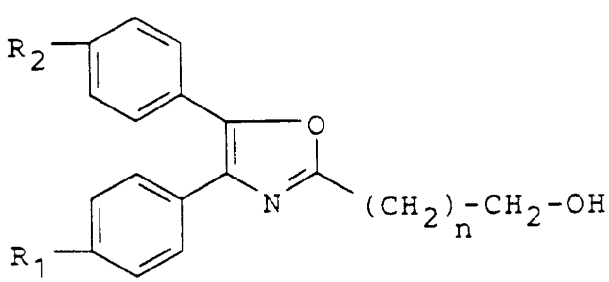

(X)

dans laquelle $R_1$, $R_2$ et n sont définis comme précédemment.

L'oxydation peut être mise en oeuvre par toute méthode connue qui n'affecte pas le reste de la molécule. Notamment on opère en présence de chlorochromate de pyridinium dans un solvant chloré (chlorure de méthylène par exemple), à une température comprise entre 0 et 20°C.

L'alcool de formule générale (X) peut être préparé par saponification de l'ester de formule générale :

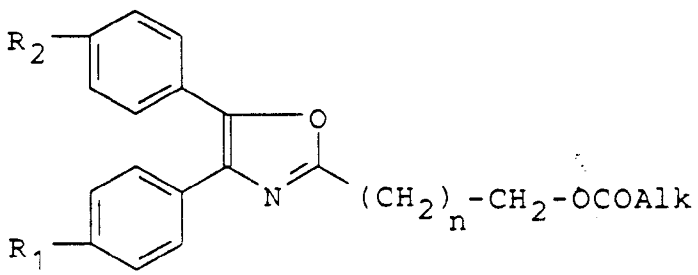

(XI)

dans laquelle $R_1$, $R_2$ et n sont définis comme précédemment et Alk est un radical alcoyle contenant 1 à 4 atomes de carbone en chaîne droite ou ramifiée. La saponification s'effectue en milieu alcoolique (méthanol, éthanol), à une température voisine de 20°C, par action de l'ammoniaque concentré, de la soude diluée ou d'un carbonate alcalin.

L'ester de formule générale (XI) peut être obtenu à partir d'un dérivé bromé de formule générale (III) par action d'un acétate alcalin en milieu acétique.

La cétone de formule générale (IX) pour laquelle R est un radical alcoyle, peut être préparée à partir d'un β-cétoester de formule générale :

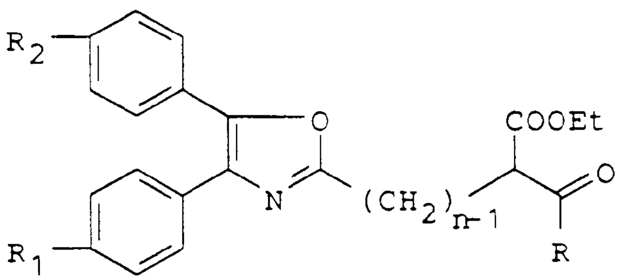

(XII)

dans laquelle $R_1$, $R_2$, R et n sont définis comme précédemment.

La réaction s'effectue en milieu acide (acide chlorhydrique par exemple), à une température comprise entre 30 et 150°C.

Le β-cétoester de formule générale (XII) peut être préparé par action de l'acétyl-acétate d'éthyle sur un dérivé bromé de formule générale :

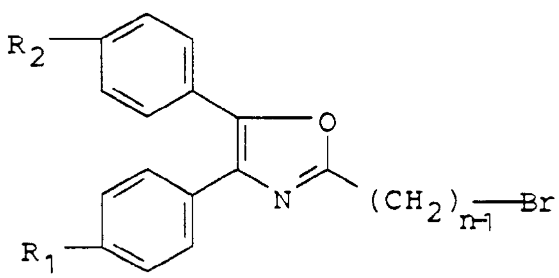

(XIII)

dans laquelle $R_1$, $R_2$ et n sont définis comme précédemment.

La réaction s'effectue généralement dans un solvant tel que le diméthylformamide à partir du dérivé sodé de l'acétylacétate d'éthyle, à une température comprise entre 30 et 110°C.

Le dérivé bromé de formule générale (XIII) peut être préparé par analogie avec la préparation du dérivé bromé de formule générale (III).

Les produits selon l'invention peuvent être purifiés par cristallisation ou par chromatographie.

Le cas échéant les isomères des produits selon l'invention peuvent être séparés selon les méthodes habituelles qui n'altèrent pas le reste de la molécule. Par exemple ils peuvent être séparés par chromatographie sur une colonne chirale.

Les produits selon la présente invention peuvent être transformés en sels métalliques selon les méthodes connues en soi. Ces sels peuvent être obtenus par action d'une base métallique forte sur un produit selon l'invention dans un solvant approprié. Le sel formé précipite après concentration éventuelle de sa solution, il est séparé par filtration, décantation ou lyophilisation.

Comme exemples de sels pharmaceutiquement acceptables, peuvent être cités les sels avec les métaux alcalins (sodium, potassium, lithium).

Les nouveaux dérivés de l'oxazole de formule générale (I) et leurs sels sont particulièrement intéressants dans le domaine de l'inflammation où interviennent le leucotriène $B_4$, et/ou la 5-lipoxygénase notamment dans le domaine ostéoarticulaire. Du fait de leur affinité pour les récepteurs à leucotriène $B_4$, ils interfèrent avec cet agoniste en bloquant son action au niveau du récepteur.

Ils interviennent également en inhibant, au niveau de la 5-lipoxygénase, la production de différents produits tels que le leucotriène $A_4$, le leucotriène B4, le leucotriène C4.

Leur affinité pour les récepteurs à leucotriène $B_4$ a pu être mise en évidence en mesurant leur effet vis à vis du binding à leucotriène $B_4$ tritié sur des neutrophiles sanguins humains selon une technique inspirée de la méthode de A. H. LIN et coll., Prostaglandins, 28, 837 (1984). Dans cette technique les produits selon l'invention se sont montrés actifs à des valeurs de Ki comprises entre 20 et 500 nM.

Leur propriété inhibitrice vis à vis de la 5-lipoxygénase a été mesurée vis à vis d'un sonicat de "Rat Basophil leukemia de type I" (RBL1) en utilisant une technique inspirée de M.M. Steinhoff et coll., Biochem. Biophysica Acta, 618, 28 (1980) et B.A. Jakshik, J. Biol. Chem., 257, 5346 (1982), et/ou sur PMN (polymorphonuclear) de rats provenant d'un extrudat péritonéal en utilisant une technique inspirée de H. Safayhi et coll., Biochem. Pharmacol., 34, 2691 (1985).

Dans ces techniques, les produits selon l'invention se sont montrés actifs à des concentrations comprises entre 20 et 500 nM ($CI_{50}$).

De plus les produits selon l'invention présentent l'intérêt d'être très faiblement toxiques. Leur toxicité ($DL_{50}$) chez la souris est comprise entre 500 mg/kg et des valeurs supérieures à 1 g/kg par voie orale.

Les exemples suivants donnés à titre non limitatif illustrent la présente invention.

## EXEMPLE 1

On chauffe 4 heures au reflux le mélange de 15 g d'oxalate acide de N-{[bis(méthoxy-4 phényl)-4,5 oxazolyl-2]-6 hexyl} hydroxylamine, 5,5 g de cyanate de potassium, 61 $cm^3$ de tétrahydrofuranne et 6 $cm^3$ d'eau. Après refroidissement, on ajoute 200 $cm^3$ d'eau et 600 $cm^3$ d'acétate d'éthyle. La phase organique est décantée, lavée avec 5 fois 50 $cm^3$ d'eau, 100 $cm^3$ d'une solution saturée de chlorure de sodium, séchée sur sulfate de magnésium, filtrée et concentrée à environ 150 $cm^3$. On essore et sèche le solide formé. On obtient 8,9 g (65%) de N-{[bis(méthoxy-4 phényl)-4,5 oxazolyl-2]-6 hexyl} N-hydroxyurée, sous forme de cristaux blancs fondant à 127°C.

L'oxalate acide de N-{[bis(méthoxy-4 phényl)-4,5 oxazolyl-2]-6 hexyl} hydroxylamine est préparé de la manière suivante :

a) Le {[bis(méthoxy-4 phényl)-4,5 oxazolyl-2]-6 hexyl}-2 dihydro-2,5 méthyl-3 oxo-5 isoxazolecarboxylate-4 d'éthyle peut être préparé comme suit:

Selon le procédé décrit par G. DOLESCHALL, on chauffe sous agitation, pendant 20 heures à 105°C, 44 g de sel de sodium du méthyl-3 hydroxy-5 isoxazolecarboxylate-4 d'éthyle en suspension dans 220 $cm^3$ de diméthylformamide avec 97 g de bis(méthoxy-4 phényl)-4,5 (bromo-6 hexyl)-2 oxazole et 2 g d'iodure de potassium. Après refroidissement, la solution contenant en suspension le bromure de sodium, est versée dans 1 litre d'eau. Le mélange est extrait par 500 $cm^3$ d'acétate d'éthyle. La phase organique est séparée par décantation, lavée par 4 fois 100 $cm^3$ d'une solution aqueuse saturée en bicarbonate de sodium, 3 fois 100 $cm^3$ d'eau et par 2 fois 100 $cm^3$ d'une solution aqueuse saturée en chlorure de sodium. Après séchage sur sulfate de magnésium, le solvant est évaporé sous pression réduite (5,2 kPa). Le résidu est chromatographié sur une colonne de 4,4 cm de diamètre intérieur, contenant 300 g de gel de silice (50 à 200 $\mu$). On élue, en recueillant des fractions de 250 $cm^3$, par 2 litres d'oxyde de diisopropyle puis par 1 litre d'un mélange d'oxyde de diisopropyle-acétate d'éthyle (80-20 en volume) et enfin par 3 litres d'oxyde de diisopropyle-acétate d'éthyle (50-50 en volume). Les fractions du dernier mélange éluant sont réunies et concentrées à sec. On obtient ainsi 101 g (86%) de {[bis(méthoxy-4 phényl)-4,5 oxazolyl-2]-6 hexyl}-2 dihydro-2,5 méthyl-3 oxo-5 isoxazolecarboxylate-4 d'éthyle sous la forme d'une huile jaunâtre directement utilisée dans la réaction ultérieure.

Spectre de RMN du proton (200 MHz, $CDCl_3$, $\delta$ en ppm J en Hz) :

1,32 (t, J=7, $-O-CH_2-CH_3$);

2,47 (s, $-CH_3$);

2,78 (t, J=7, $-N=C-CH_2-$);

3,78 (2s, $-OCH_3$);

3,83 (t, J=7, $-CH_2N<$);

4,27 (q, J=7, $-O-CH_2-CH_3$).

b) On chauffe sous agitation, pendant 20 heures à 95°C, 101 g de {[bis (méthoxy-4 phényl)-4,5 oxazolyl-2]-6 hexyl}-2 dihydro-2,5 méthyl-3 oxo-5 isoxazolecarboxylate-4 d'éthyle en solution dans 190 $cm^3$ d'acide acétique pur avec 380 $cm^3$ d'acide chlorhydrique 6N.

Le mélange est alors ramené à 60°C puis concentré à sec sous pression réduite (5,2 kPa). Le résidu est dissous dans 1 litre d'eau et la solution lavée avec 0,5 litre d'oxyde de diéthyle. La solution aqueuse décantée est additionnée de 200 $cm^3$ de soude 2 fois normale et extraite avec 3 fois 200 $cm^3$ d'oxyde de diéthyle. Les extraits éthérés réunis sont lavés avec 100 $cm^3$ d'une solution saturée en chlorure de sodium, séchés sur sulfate de magnésium, filtrés, et le solvant évaporé. Le résidu, en solution dans 100 $cm^3$ de dichlorométhane, est salifié par addition de 14 g d'acide oxalique anhydre. La solution est concentrée à sec sous pression réduite (5,2 kPa). Le solide résultant, repris par 300 $cm^3$ d'acétate d'éthyle, est essoré, lavé avec 200 $cm^3$ d'oxyde de diéthyle, et séché. On obtient 52 g (56%) d'oxalate acide de N-{[bis(méthoxy-4 phényl)-4,5 oxazolyl-2]-6 hexyl} hydroxylamine, sous la forme d'une poudre blanche fondant à 122°C.

Le bis (méthoxy-4 phényl)-4,5 (bromo-6 hexyl)-2 oxazole peut être préparé de la manière suivante:

On chauffe sous agitation, pendant 4 heures à 80°C, 223 g de bis(méthoxy-4 phényl)-1,2 (bromo-7 heptanamido)-2 éthanone dans 430 $cm^3$ de toluène avec 222 g d'oxychlorure de phosphore. Après concentration sous pression réduite (6 kPa), le résidu est additionné de 500 $cm^3$ d'eau glacée et agité avec 1 litre d'oxyde de diéthyle. La phase organique est décantée, lavée par 3 fois 300 $cm^3$ d'eau, 2 fois 100 $cm^3$ d'une solution saturée en bicarbonate de sodium, 2 fois 100 $cm^3$ d'une solution saturée en chlorure de sodium, séchée sur sulfate de magnésium, filtrée et concentrée à sec sous pression réduite (5,2 kPa). Le résidu est chromatographié sur une colonne de 4,4 cm de diamètre contenant 300 g de gel de silice (50 à 200 $\mu$). On élue, par 2 litres d'oxyde de diisopropyle en recueillant des fractions de 200 $cm^3$ On concentre à sec les fractions comprises entre 0,5 litre et 2 litres. On obtient ainsi 184,5 g (86%) de bis(méthoxy-4 phényl)-4,5 (bromo-6 hexyl)-2 oxazole, sous la forme d'une huile jaunâtre directement utilisable dans la réaction ultérieure.

Spectre de RMN du proton (200 MHz, $CDCl_3$, $\delta$ en ppm, J en Hz) :

2,84 (t, J=7,5, $-N=C-CH_2-$);

3,42 (t, J=7, $-CH_2Br$);

3,82 (2s, $-OCH_3$).

La bis (méthoxy-4 phényl)-1,2 (bromo-7 heptanamido)-2 éthanone peut être préparée de la manière suivante:

A une suspension, refroidie à 5°C et agitée, de 154 g de chlorhydrate d'amino-2 bis(méthoxy-4 phényl)-1,2 éthanone (obtenu selon G.DREFAHL et M.HARTMAN, Ann. Chem., 589, 82-90 (1954)) dans 600 $cm^3$ de dichlorométhane contenant 119,5 g de chlorure de l'acide bromo-7 heptanoïque (préparé selon R.C. ELDERFIELD, et coll., Croat. Chem Acta, 35, 85-91 (1963)), on ajoute en 2 heures une solution de 99 g de pyridine dans 125 $cm^3$ de dichlorométhane. On agite le mélange 20 heures à la température ambiante. On ajoute 150 $cm^3$ d'eau, décante la phase organique qui est lavée avec 2 fois 100 $cm^3$ d'acide chlorhydrique normal puis à l'eau (2 fois 60 $cm^3$). On sèche sur sulfate de magnésium, filtre et évapore le solvant sous pression réduite (0,2 kPa). On obtient 223 g (96%) de bis (méthoxy-4 phényl)-1,2 (bromo-7 heptanamido)-2 éthanone, sous la forme d'une huile jaunâtre directement utilisée dans l'étape ultérieure.

Spectre de RMN du proton (200 MHz, $CDCl_3$, $\delta$ en ppm, J en Hz)

2,22 (t, J=7,5 , $O=C-CH_2-$);

3,34 (t, J=7, $-CH_2-Br$);

3,71 et 3,79 (2s, deux $-OCH_3$);

6,47 (d, J=7, -CO-CH-N);

7,05 (d, J=7, -CONH-).

## EXEMPLE 2

La N-{[bis(méthoxy-4 phényl)-4,5 oxazolyl-2]-5 pentyl} N-hydroxyurée peut être préparée de la manière suivante :

A une solution de 3,45 g de N-{[bis(méthoxy-4 phényl)-4,5 oxazolyl-2]-5 pentyl} hydroxylamine en solution dans 18 $cm^3$ de tétrahydrofuranne et 2 $cm^3$ d'eau, on ajoute sous agitation 0,82 g d'acide oxalique puis 1,61 g de cyanate de potassium et chauffe au reflux pendant 4 heures. Après refroidissement, on ajoute 20 $cm^3$ d'eau et 100 $cm^3$ d'acétate d'éthyle. La phase organique est décantée, lavée avec 5 fois 20 $cm^3$ d'eau, 50 $cm^3$ de solution saturée en chlorure de sodium, séchée sur sulfate de magnésium, filtrée, évaporée à sec. On chromatographie le résidu de l'évaporation sur une colonne de 1,6 cm de diamètre, contenant 50 g de gel de silice (50 à 200 $\mu$). On élue, en recueillant des fractions de 100 $cm^3$, par 1,8 litre d'acétate d'éthyle. On concentre à sec sous pression réduite (0,2 kPa) les fractions comprises entre 0,5 et 1,8 litre. On obtient 1,15 g (30%) de N-{[bis(méthoxy-4 phényl)-4,5 oxazolyl-2]-5 pentyl} N-hydroxyurée, sous la forme d'un solide jaunâtre fondant

à 60°C.

On peut préparer la N-{[bis(méthoxy-4 phényl)-4,5 oxazolyl-2]-5 pentyl} hydroxylamine selon les conditions définies dans l'exemple 1. On traite 17,5 g de {[bis (méthoxy-4 phényl)-4,5 oxazolyl-2]-5 pentyl}-2 dihydro-2,5 méthyl-3 oxo-5 isoxazolecarboxylate-4 d'éthyle dans 100 $cm^3$ d'acide acétique par 66 $cm^3$ d'acide chlorhydrique 6N. On concentre à sec. Après traitement on purifie le produit brut de la réaction par chromatographie sur une colonne de 3 cm de diamètre, contenant 80 g de gel de silice (50 à 200 $\mu$). On élue, en recueillant des fractions de 100 $cm^3$, par 2,3 litres d'un mélange d'oxyde de diisopropyle-méthanol (95-5 en volume). On concentre à sec sous pression réduite (0,2 kPa) les fractions comprises entre 1 et 2,3 litres. On obtient 8,2 g (64%) de N-{[bis(méthoxy-4 phényl)-4,5 oxazolyl-2]-5 pentyl} hydroxylamine, sous la forme d'une huile incolore.

Spectre de RMN du proton (250 MHz, $CDCl_3$, $\delta$ en ppm, J en Hz)
2,85 (t, J=7,5, $-N=C-CH_2-$);
2,96 (t, J=7, $>N-CH_2-$);
3,84 (s, deux $-OCH_3$);
4 à 4,8 (mf, -OH).

On peut préparer le {[bis(méthoxy-4 phényl)-4,5 oxazolyl-2]-5 pentyl}-2 dihydro-2,5 méthyl-3 oxo-5 isoxazolecarboxylate-4 d'éthyle selon l'exemple 1.

A partir de 34,5 g de bis(méthoxy-4 phényl)-4,5 (bromo-5 pentyl)-2 oxazole, 16 g de sel de sodium du méthyl-3 hydroxy-5 isoxazolecarboxylate-4 d'éthyle et 0,8 g d'iodure de potassium dans 200 $cm^3$ de diméthylformamide, on obtient après traitement un résidu visqueux. On chromatographie celui-ci sur une colonne de 3,8 cm de diamètre, contenant 90 g de gel de silice (50 à 200 $\mu$). On élue, en recueillant des fractions de 150 $cm^3$, par 1,5 litre d'un mélange d'oxyde de diisopropyle-acétate d'éthyle (50-50 en volume) puis par 2,1 litres d'acétate d'éthyle. Les fractions réunies de ce dernier éluant, sont concentrées à 130 $cm^3$ et additionnées de 300 $cm^3$ d'oxyde de diisopropyle. On essore le précipité obtenu, sèche, et obtient 19 g (45%) de {[bis (méthoxy-4 phényl)-4,5 oxazolyl-2]-5 pentyl}-2 dihydro-2,5 méthyl-3 oxo-5 isoxazolecarboxylate-4 d'éthyle, sous la forme de cristaux blancs fondant à 127°C.

On peut préparer le bis(méthoxy-4 phényl)-4,5 (bromo-5 pentyl)-2 oxazole dans les conditions de l'exemple 1.

On chauffe 37,5 g de bis (méthoxy-4 phényl)-1,2 (bromo-6 hexanamido)-2 éthanone, 135 $cm^3$ de toluène et 38 g d'oxychlorure de phosphore. On isole, après traitements 34,5 g (96%) de bis(méthoxy-4 phényl)-4,5 (bromo-5 pentyl)-2 oxazole sous la forme d'une huile visqueuse directement utilisée dans l'étape ultérieure.

Spectre de RMN du proton (300 MHz, $CDCl_3$, $\delta$ en ppm, J en Hz)
2,82 (t, J=7,5, $-N=C-CH_2-$)
3,41 (t, J=7, $-CH_2-Br$)
3,79 (s, deux $-OCH_3$)

La bis (méthoxy-4 phényl)-1,2 (bromo-6 hexanamido)-2 éthanone peut être préparée comme dans l'exemple 1.

A partir de 64,6 g de chlorhydrate d'amino-2 bis(méthoxy-4 phényl)-1,2 éthanone (obtenu selon G.DREFAHL et M.HARTMAN, Ann.Chem., 589, p.82-90 (1954)) dans 250 $cm^3$ de dichlorométhane contenant 47 g de chlorure de l'acide bromo-6 hexanoïque, on ajoute en 50 minutes une solution de 41,5 g de pyridine dans 50 $cm^3$ de dichlorométhane. On agite le mélange 20 heures à la température ambiante. On ajoute 60 $cm^3$ d'eau, décante la phase organique qui est lavée avec 2 fois 50 $cm^3$ d'acide chlorhydrique normal, puis à l'eau (2 fois 30 $cm^3$). On sèche sur sulfate de magnésium, filtre et évapore le solvant sous pression réduite (0,2 kPa). Le résidu solide est recristallisé dans un mélange de 80 $cm^3$ d'isopropanol et de 1300 $cm^3$ d'oxyde de diisopropyle. On obtient 84,9 g (90%) de bis (méthoxy-4 phényl)-1,2 (bromo-6 hexanamido)-2 éthanone sous la forme de cristaux blancs fondant à 93°C.

EXEMPLE 3

On chauffe 4 heures au reflux un mélange de 1,8 g d'oxalate acide de N-{[bis(chloro-4 phényl)-4,5 oxazolyl-2]-6 hexyl} hydroxylamine, 0,65 g de cyanate de potassium, dans 10 $cm^3$ de tétrahydrofuranne et 1 $cm^3$ d'eau. Après refroidissement, on ajoute 30 $cm^3$ d'eau et 100 $cm^3$ d'acétate d'éthyle. La phase organique est décantée, lavée avec 3 fois 20 $cm^3$ d'eau, 20 $cm^3$ d'une solution saturée en chlorure de sodium, séchée sur sulfate de magnésium, filtrée et concentrée sous pression réduite (5,2 kPa). L'huile obtenue cristallise dans 25 $cm^3$ de diéthyle oxyde. Le précipité est essoré et recristallisé dans 12 $cm^3$ d'acétate d'isopropyle, et on obtient 1,1 g (67%) de N-{[bis (choro-4 phényl)-4,5 oxazolyl-2]-6 hexyl} N-hydroxyurée, sous la forme de cristaux blancs fondant à 124°C.

On peut préparer l'oxalate acide de N-{[bis(chloro-4 phényl)-4,5 oxazolyl-2]-6 hexyl} hydroxylamine selon

les conditions définies dans l'exemple 1.

On traite 11 g de {[bis(chloro-4 phényl)-4,5 oxazolyl-2]-6 hexyl}-2 dihydro-2,5 méthyl-3 oxo-5 isoxazolecarboxylate-4 d'éthyle dans 20 $cm^3$ d'acide acétique par 40 $cm^3$ d'acide chlorhydrique 6N. On concentre à sec. Après traitement on purifie le produit brut de la réaction par chromatographie sur une colonne de 2 cm de diamètre, contenant 50 g de gel de silice (50 à 200 μ). On élue, par 0,75 litre d'un mélange d'oxyde de diisopropyle-acétate d'éthyle (50-50 en volume) en recueillant des fractions de 25 $cm^3$. On concentre à sec sous pression réduite (0,2 kPa) les fractions comprises entre 0,25 et 0,75 litre. On obtient 6 g d'une huile jaune pâle. A la solution de celle-ci dans 30 $cm^3$ d'acétate d'éthyle, on ajoute une solution de 1,35 g d'acide oxalique dans 20 $cm^3$ d'acétate d'éthyle. On précipite le sel attendu par addition de 100 $cm^3$ d'oxyde de diisopropyle. Le solide est essoré et séché. On obtient 5 g (50%) d'oxalate acide de N-{[bis(chloro-4 phényl)-4,5 oxazolyl-2]-6 hexyl} hydroxylamine, sous la forme de cristaux blancs fondant à 165°C.

On peut préparer le {[bis (choro-4 phényl)-4,5 oxazolyl-2]-6 hexyl}-2 dihydro-2,5 méthyl-3 oxo-5 isoxazolecarboxylate-4 d'éthyle selon l'exemple 1.

A partir de 13,5 g de bis(chloro-4 phényl)-4,5 (bromo-6 hexyl)-2 oxazole, 6 g de sel de sodium du méthyl-3 hydroxy-5 isoxazolecarboxylate-4 d'éthyle et 1 g d'iodure de potassium dans 80 $cm^3$ de diméthylformamide. On recristallise le produit issu de la réaction dans 150 $cm^3$ d'acétate d'isopropyle. On obtient 11 g (68%) d'une poudre blanche fondant à 115°C.

On peut préparer le bis(chloro-4 phényl)-4,5 (bromo-6 hexyl)-2 oxazole de la manière suivante :

On chauffe 4 heures à 90°C, 18 g de bis (chloro-4 phényl)-1,2 (bromo-7 heptanamido)-2 éthanone en solution dans 150 $cm^3$ de toluène et 12,8 g de tribromure de phosphore. A température ambiante, on additionne 150 $cm^3$ d'acétate d'éthyle, 150 $cm^3$ d'eau et 100 $cm^3$ d'une solution de soude 2N. On agite et décante la phase organique. On lave celle-ci par 3 fois 50 $cm^3$ d'eau, 40 $cm^3$ d'une solution saturée en chorure de sodium, sèche sur sulfate de magnésium, filtre et concentre sous pression réduite (5,2 kPa). On chromatographie le produit de la réaction, sur une colonne de 3 cm de diamètre, contenant 100 g de gel de silice (50 à 200 μ). On élue, en recueillant des fractions de 50 $cm^3$, par 0,8 litre d'un mélange d'oxyde de diisopropyle-hexane (50-50 en volume). Les fractions comprises entre 0,5 et 0,8 litre sont concentrées à sec sous pression réduite (0,2 kPa). Le résidu huileux obtenu cristallise dans 100 $cm^3$ d'éther de pétrole. On obtient 13,5 g (78%) de cristaux blancs fondant à 30°C.

On peut préparer la bis (chloro-4 phényl)-1,2 (bromo-7 heptanamido)-2 éthanone selon l'exemple 1.

A partir de 15,8 g de chlorhydrate d'amino-2 bis(chloro-4 phényl)-1,2 éthanone (obtenu selon G.DREFAHL et coll., Ann. Chem., 589, 82-90 (1954)) dans 100 $cm^3$ de dichlorométhane contenant 12 g de chlorure de l'acide bromo-7 heptanoïque, on ajoute 10 g de pyridine dans 12 $cm^3$ de dichlorométhane. On extrait et cristallise le résidu solide obtenu dans 200 $cm^3$ d'oxyde de diisopropyle. On isole 19 g (80%) de cristaux blancs fondant à 98°C.

## EXEMPLE 4

La N-{[bis(chloro-4 phényl)-4,5 oxazolyl-2]-7 heptyl}-2 N-hydroxyurée (R,S) peut être préparée de la manière suivante:

On dissout 2,6 g de N-{[bis(chloro-4 phényl)-4,5 oxazolyl-2]-7 heptyl-2} hydroxylamine (R,S) dans 50 $cm^3$ de tétrahydrofuranne contenant 6,8 $cm^3$ d'acide chlorhydrique normal. On ajoute à la solution 0,55 g de cyanate de potassium et agite le mélange 5 heures au reflux. Après évaporation à sec, on ajoute 150 $cm^3$ d'oxyde de diéthyle et 30 $cm^3$ d'eau. On lave la solution éthérée avec 3 fois 30 $cm^3$ d'eau, 30 $cm^3$ d'une solution saturée en chlorure de sodium, sèche sur sulfate de magnésium et concentre à sec. On chromatographie le résidu sur une colonne de 2 cm de diamètre contenant 20 g de gel de silice (50 à 200μ). On élue avec 1,2 litre d'acétate d'éthyle, en recueillant des fractions de 100 $cm^3$. On concentre à sec les fractions comprises entre 0,4 et 1,2 litre et obtient 1,7 g d'une huile incolore. Cette dernière cristallise dans 30 $cm^3$ d'un mélange d'oxyde de diéthyle-oxyde de diisopropyle (35-65 en volume). On obtient 1,5 g (52%) de cristaux blancs fondant à 125°C.

Selon l'exemple 7, on peut préparer la N-{[bis(chloro-4 phényl)-4,5 oxazolyl-2]-7 heptyl-2} hydroxylamine (R,S) comme suit:

On chauffe un mélange de 11 g de bis(chloro-4 phényl)-4,5 (bromo-4 butyl)-2 oxazole en solution dans 30 $cm^3$ de diméthylformamide, et du dérivé sodé de l'acétylacétate d'éthyle, obtenu à partir de 7,5 g d'acétylacétate d'éthyle, dans 40 $cm^3$ de diméthylformamide, 1,4 g d'hydrure de sodium à 50% et 0,1 g d'iodure de potassium. On purifie le produit issu de la réaction par chromatographie sur une colonne de 3,8 cm de diamètre, contenant 200 g de gel de silice (50 à 200μ). On élue, en recueillant des fractions de 200 $cm^3$, avec 3,6 litres d'un mélange à parties égales d'oxyde de diisopropyle et d'hexane. On concentre à sec les fractions comprises entre 1,6 et 3,6 litres. On obtient 8,2 g d'une huile jaunâtre. On chauffe celle-ci dans 90 $cm^3$ d'acide chlorhy-

drique 5N et 45 $cm^3$ d'acide acétique. On chromatographie le produit de la réaction sur une colonne de 3 cm de diamètre, contenant 120 g de gel de silice (50 à 200 μ). On élue, en recueillant des fractions de 100 $cm^3$, par 1,5 litre d'oxyde de diisopropyle. On concentre à sec les fractions comprises entre 0,6 et 1,5 litre. On obtient 6,2 g (89%) d'une huile incolore. On dissout celle-ci dans 70 $cm^3$ d'éthanol, ajoute une solution de 2,7 g de chlorhydrate d'hydroxylamine dans 6 $cm^3$ d'eau, et 6,6 g de pyridine. On chauffe ce mélange 6 heures à reflux. Après traitement, on obtient 6,4 g d'une huile jaune pâle. On dissout cette dernière dans 70 $cm^3$ d'acide acétique et on ajoute 1,4 g de cyanoborhydrure de sodium. Le produit de la réaction est chromatographié sur une colonne de 2 cm de diamètre, contenant 35 g de gel de silice (50 à 200 μ). On élue, en recueillant des fractions de 100 $cm^3$, par 0,3 litre d'oxyde de diisopropyle, puis par 1,1 litre d'un mélange d'oxyde de diisopropyle-méthanol (98-2 en volume). La concentration à sec des fractions de ce dernier mélange donne 5,7 g (90%) de N-{[bis(chloro-4 phényl)-4,5 oxazolyl-2]-7 heptyl-2} hydroxylamine (R,S) sous la forme de cristaux blancs fondant à 90°C.

On peut préparer le bis(chloro-4 phényl)-4,5 (bromo-4 butyl)-2 oxazole selon les conditions définies dans l'exemple 7. A une suspension de 20 g de chlorhydrate d'amino-2 bis(chloro-4 phényl)-1,2 éthanone dans 150 $cm^3$ de dichlorométhane contenant 13,5 g de chlorure de bromo-5 pentanoyl, on ajoute 11,2 g de pyridine dans 12 $cm^3$ de dichlorométhane. On recristallise le résidu solide obtenu dans 75 $cm^3$ d'oxyde de diisopropyle. On isole 20 g (71%) de bis(chloro-4 phényl)-1,2 (bromo-5 pentanamido)-2 éthanone, cristaux blancs fondant à 102°C.

On chauffe sous agitation pendant 4 heures à 90°C, 20 g de bis(chloro-4 phényl)-1,2 (bromo-5 pentanamido)-2 éthanone dans 150 $cm^3$ de toluène, avec 14,5 g de tribromure de phosphore. A température ambiante, on additionne 100 g de glace, 150 $cm^3$ d'oxyde de diéthyle et 100 $cm^3$ d'une solution de soude 2N. On agite 0,5 heure et décante la phase organique. On lave celle-ci par 3 fois 50 $cm^3$ d'eau, 40 $cm^3$ d'une solution saturée en chlorure de sodium, sèche sur sulfate de magnésium, filtre et concentre sous pression réduite (5,2 kPa). On chromatographie le produit de la réaction sur une colonne de 3,8 cm de diamètre, contenant 200 g de gel de silice (50 à 200 μ). On élue, en recueillant des fractions de 150 $cm^3$, avec 1,8 litre d'un mélange d'oxyde de diisopropyle-hexane (50-50 en volume). On concentre à sec sous pression réduite (5,2 kPa) les fractions comprises entre 0,6 et 1,8 litre. Le résidu solide est recristallisé dans 100 $cm^3$ d'hexane. On obtient 11 g (57%) de bis(chloro-4 phényl)-4,5 (bromo-4 butyl)-2 oxazole, sous la forme d'une poudre jaune fondant à 79°C.

## EXEMPLE 5

On agite à 110°C pendant 3 heures, 27 g d'oxalate acide de N-{[bis(méthoxy-4 phényl)-4,5 oxazolyl-2]-6 hexyl} hydroxylamine dans 100 $cm^3$ d'acide acétique contenant 9,5 g d'acétate de sodium et 23 g d'anhydride acétique. Après refroidissement, on ajoute 150 $cm^3$ d'eau, agite la solution pendant 1 heure à 20°C et concentre à sec sous pression réduite (5,2 kPa). On dissout le résidu organominéral dans 200 $cm^3$ d'acétate d'éthyle et 50 $cm^3$ d'eau, sépare la phase organique et lave celle ci avec 3 fois 50 $cm^3$ d'eau, 50 $cm^3$ d'une solution saturée en chlorure de sodium, sèche sur sulfate de magnésium, filtre et concentre à sec. Le résidu huileux (23 g) est chromatographié sur une colonne de 2 cm de diamètre contenant 150 g de gel de silice (50 à 200 μ). On élue avec 1 litre d'oxyde de diisopropyle en recueillant des fractions de 50 $cm^3$. Les fractions comprises entre 0,15 et 1 litre sont concentrées à sec. On obtient 17 g (64%) de N-acétoxy N-{[bis(méthoxy-4 phényl)-4,5 oxazolyl-2]-6 hexyl} acétamide.

Spectre de RMN du proton (250 MHz, $CDCl_3$, δ en ppm, J en Hz) :
2,et 2,16 (2s, $CH_3CO-N-OCOCH_3$);
2,82 (t, J=7,5, $-N=C-CH_2-$);
3,68 (t, J=7, $>N-CH_2-$);
3,82 (s, deux $-OCH_3$).

On dissout l'acétamide obtenu ci-dessus, dans 200 $cm^3$ de méthanol contenant 100 $cm^3$ d'ammoniaque à 33%. On agite le mélange pendant 2 heures à 20°C. Après concentration à sec sous pression réduite, on ajoute 50 $cm^3$ d'eau, extrait avec de l'oxyde de diéthyle (3 fois 150 $cm^3$) et lave les solutions éthérées réunies avec 100 $cm^3$ de solution saturée en chlorure de sodium. On sèche sur sulfate de magnésium, filtre et élimine le solvant. On chromatographie le résidu de l'évaporation sur une colonne de 2 cm de diamètre contenant 150 g de gel de silice (50 à 200 μ). On élue par 2,5 litres d'acétate d'éthyle, en recueillant des fractions de 50 $cm^3$. On concentre à sec sous pression réduite (0,2 kPa) les fractions comprises entre 1 et 1,5 litre. On obtient ainsi 10 g (64,5%) d'acide N-{[bis(méthoxy-4 phényl)-4,5 oxazolyl-2]-6 hexyl} acétohydroxamique, sous la forme d'une huile jaunâtre.

Spectre de RMN du proton (200 MHz, DMSO d6, δ en ppm et J en Hz):
2 (s, $-COCH_3$);
2,78 (t, J=7,5, $-N=C-CH_2-$);

3,5 (t, J=7,5, >N-$CH_2$-);
3,79 et 3,81 (2s, deux -$OCH_3$);
9,7 (s, >N-OH).

EXEMPLE 6

Au mélange, agité à 0°C, de 4 g d'oxalate acide de N-{[bis(méthoxy-4 phényl)-4,5 oxazolyl-2]-6 hexyl} hydroxylamine, 20 $cm^3$ de soude 2N, 25 $cm^3$ de dichlorométhane, on ajoute 1 g de chorure d'isobutyryle. Après 2 heures à basse température puis 2 heures à 20°C, on ajoute 100 $cm^3$ d'acétate d'éthyle; la phase organique est décantée, lavée avec 2 fois 20 $cm^3$ d'eau, 30 $cm^3$ d'acide chlorhydrique normal, 2 fois 30 $cm^3$ d'une solution saturée en chlorure de sodium, séchée sur sulfate de magnésium; on filtre et élimine le solvant. On chromatographie le résidu de l'évaporation sur une colonne de 1,8 cm de diamètre, contenant 25 g de gel de silice (50 à 200 $\mu$). On élue, en recueillant des fractions de 50 $cm^3$, par 0,5 litre d'oxyde de diisopropyle puis par 0,7 litre d'un mélange d'oxyde de diisopropyle-acétate d'éthyle (80-20 en volume). Les fractions du dernier mélange éluant sont réunies et concentrées à sec sous pression réduite (2kPa). On obtient 1,6 g (41%) de N-{[bis(méthoxy-4 phényl)-4,5 oxazolyl-2]-6 hexyl} N-hydroxy-isobutyramide, sous la forme d'une huile jaunâtre.

Spectre de RMN du proton (200 MHz, $CDCl_3$, $\delta$ en ppm et J en Hz) :
2,75 et 3,1 (2 mt, >CH- des 2 rotamères);
2,85 (t, J=7,5, -N=C-$CH_2$-);
3,65 (t, J=7,5, >N-$CH_2$-);
3,83 (s, deux -$OCH_3$);
8,5 et 9,2 (2 mf N-OH des deux rotamères).

EXEMPLE 7

On agite à 110°C pendant 3 heures, 6,3 g de N-{[bis(méthoxy-4 phényl)-4,5 oxazolyl-2]-7 heptyl-2} hydroxylamine (R,S) dans 50 $cm^3$ d'acide acétique contenant 6,2 g d'anhydride acétique. Après refroidissement, on ajoute 50 $cm^3$ d'eau, agite la solution pendant 1 heure à 20°C et concentre à sec sous pression réduite (5,2 kPa). On dissout le résidu organique dans 200 $cm^3$ d'oxyde de diéthyle et 50 $cm^3$ d'eau, sépare la phase organique et lave celle-ci avec 5 fois 50 $cm^3$ d'eau, 30 $cm^3$ d'une solution saturée en chlorure de sodium, sèche sur sulfate de magnésium, filtre et concentre à sec. Le résidu huileux (7,5 g) est chromatographié sur une colonne de 3 cm de diamètre, contenant 80 g de gel de silice (50 à 200 $\mu$). On élue par 1,5 litre d'un mélange d'oxyde de diisopropyle-méthanol (98-2 en volume), en recueillant des fractions de 100 $cm^3$. On concentre à sec les fractions comprises entre 0,6 et 1,5 litre et on obtient 7 g (92%) de N-acétoxy N-{[bis(méthoxy-4 phényl)-4,5 oxazolyl-2]-7 heptyl-2} acétamide, sous la forme d'une huile incolore.

Spectre de RMN du proton (300 MHz, $CDCl_3$, $\delta$ en ppm et J en Hz à 300°K);
1,18 (d, J=7, -$CH_3$);
2 et 2,17 (2s, $CH_3CO$-N-$OCOCH_3$);
2,82 (t, J=7,5, -N=C-$CH_2$-);
3,85 (s, deux -$OCH_3$);
4,5 (mt, >N-CH<).

On dissout 6,6 g de l'acétamide obtenu ci-dessus, dans 50 $cm^3$ de méthanol contenant 20 $cm^3$ d'ammoniaque à 33%. On agite le mélange pendant 2 heures à 20°C. Après évaporation à sec sous pression réduite, on ajoute 30 $cm^3$ d'eau, extrait avec de l'oxyde de diéthyle (2 fois 150 $cm^3$) et lave les solutions éthérées réunies avec 4 fois 40 $cm^3$ d'eau, 30 $cm^3$ d'une solution saturée en chlorure de sodium; on sèche sur sulfate de magnésium, filtre et concentre à sec. Le résidu huileux est chromatographié sur une colonne de 3 cm de diamètre, contenant 90 g de gel de silice (50 à 200 $\mu$). On élue, en recueillant des fractions de 100 $cm^3$, par 1,2 litre d'acétate d'éthyle. On concentre à sec sous pression réduite (0,2 kPa) les fractions comprises entre 0,3 et 1,2 litre et on obtient 5,7 g (94%) d'acide N-{[bis(méthoxy-4 phényl)-4,5 oxazolyl-2]-7 heptyl-2} acétohydroxamique (R,S) sous la forme d'une huile incolore.

Spectre de RMN du proton (400 MHz, DMSO d6, $\delta$ en ppm et J en Hz) :
1,03 (d, J=7, -$CH_3$);
1,98 (s, -$COCH_3$);
2,77 (t, J=7,5, -N=C-$CH_2$-);
3,77 et 3,8 (2s, deux -$OCH_3$);
4,43 (mt,>N-CH<);
9,3 (s, >N-OH).

L'oxalate acide de la N-{[bis(méthoxy-4 phényl)-4,5 oxazolyl-2]-7 heptyl-2} hydroxylamine (R,S) peut être

préparé de la manière suivante:

On agite à 10°C une solution de 9,9g de [bis(méthoxy-4 phényl)-4,5 oxazolyl-2]-7 hydroxyimino-2 heptane dans 110 $cm^3$ d'acide acétique et on ajoute en 20 minutes 2,1 g de cyanoborohydrure de sodium. Après 2 heures à cette température, le mélange est traité par 200 g de glace, 200 $cm^3$ de soude 10N puis extrait avec 2 fois 200 $cm^3$ d'oxyde de diéthyle. Les phases organiques réunies sont lavées par 4 fois 50 $cm^3$ d'eau, 40 $cm^3$ d'une solution saturée en chlorure de sodium, et séchées sur sulfate de magnésium. Le solvant est évaporé sous pression réduite (5,2 kPa). Le résidu de l'évaporation est chromatographié sur une colonne de 2 cm de diamètre, contenant 100 g de gel de silice (50 à 200 μ). On élue par un mélange d'oxyde de diisopropyle-méthanol (98-2 en volume), puis par 1,4 litre d'un mélange d'oxyde de diisopropyle-méthanol (95-5 en volume) en recueillant des fractions de 100 $cm^3$. L'évaporation des fractions de ce dernier mélange donne 7,7 g (85%) de N-{[bis(méthoxy-4 phényl)-4,5 oxazolyl-2]-7 heptyl-2} hydroxylamine (R,S) sous la forme d'une huile visqueuse et incolore.

A une solution agitée de 1,4 g du dérivé de l'hydroxylamine obtenue ci-dessus dans 40 $cm^3$ de dichlorométhane, on ajoute 0,3 g d'acide oxalique. La solution résultante est concentrée sous pression réduite (5,2 kPa), puis le résidu solide dissous dans 50 $cm^3$ d'acétate d'éthyle. Le sel formé est précipité de sa solution dans l'acétate d'éthyle par addition de 100 $cm^3$ d'oxyde de diisopropyle. Le solide est éssoré et séché. On obtient 1,4 g (82%) d'oxalate acide de N-{[bis(méthoxy-4 phényl)-4,5 oxazolyl-2]-7 heptyl-2} hydroxylamine (R,S) sous la forme d'une poudre blanche cristalline fondant à 90°C.

On peut préparer le (bis(méthoxy-4 phényl)-4,5 oxazolyl-2]-7 hydroxyimino-2 heptane de la manière suivante:

A une suspension de 36,7 g de chlorhydrate d'amino-2 bis(méthoxy-4 phényl)-1,2 éthanone dans 250 $cm^3$ de dichlorométhane contenant 23,8 g de chlorure de bromo-5 pentanoyl, on ajoute 21 g de pyridine dans 25 $cm^3$ de dichlorométhane, selon les conditions définies dans l'exemple 1 et on isole 47,2 g d'une huile brunâtre. On dissout celle-ci dans 250 $cm^3$ de toluène, et traite par 49 g d'oxychlorure de phosphore. On chromatographie le produit issu de la réaction sur une colonne de 5,8 cm de diamètre, contenant 400 g de gel de silice (50 à 200 μ). On élue avec 2,5 litres d'oxyde de diisopropyle, en recueillant des fractions de 250 $cm^3$. On concentre à sec sous pression réduite (5,2 kPa) les fractions comprises entre 1 et 2,5 litres. On obtient $^3$3 g (75%) de bis(méthoxy-4 phényl)-4,5 (bromo-4 butyl)-2 oxazole, sous la forme d'une huile jaunâtre.

Spectre de RMN du proton (200 MHz, $CDCl_3$, δ en ppm et J en Hz):
2,89 (t, J=7,5, $-N=C-CH_2-$);
3,47 (t, J=7,5, $-CH_2-Br$);
3,84 (s, deux $-OCH_3$).

On dissout 14 g de l'oxazole obtenu ci-dessus, dans 30 $cm^3$ de diméthylformamide et ajoute la solution au dérivé sodé de l'acétylacétate d'éthyle, obtenu à partir de 10 g d'acétylacétate d'éthyle, dans 40 $cm^3$ de diméthylformamide et 1,9 g d'hydrure de sodium à 50%. On ajoute au mélange 0,1 g d'iodure de potassium et chauffe 10 heures à 80°C. A la température ambiante on dilue avec 150 $cm^3$ d'eau glacée, extrait avec 2 fois 150 $cm^3$ d'oxyde de diéthyle. Les extraits organiques réunis sont lavés par 5 fois 40 $cm^3$ d'eau, 30 $cm^3$ d'une solution saturée en chlorure de sodium et séchés sur sulfate de magnésium. Le solvant est évaporé sous pression réduite (5,2 kPa). On chromatographie le résidu sur une colonne de 3,8 cm de diamètre, contenant 280 g de gel de silice (50 à 200 μ). On élue avec 2,5 litres d'oxyde de diisopropyle en recueillant des fractions de 100 $cm^3$. On concentre à sec les fractions comprises entre 1,2 et 2,5 litres. On obtient 9,8 g de [bis(méthoxy-4 phényl)-4,5 oxazolyl-2]-6 acétyl-2 hexanoate d'éthyle sous la forme d'une huile jaunâtre.

Spectre de RMN du proton (200 MHz, $CDCl_3$, δ en ppm et J en Hz) :
1,27 (t, J=7, $-CH_2-CH_3$);
2,24 (s, $-COCH_3$);
2,84 (t, J=7,5, $-N=C-CH_2-$);
3,45 (t, J=7,5, -OCO-CH-CO);
3,84 (s, deux $-OCH_3$);
4,2 (q, J=7,5, $-O-CH_2-CH_3$).

On dissout le dérivé de l'hexanoate d'éthyle, obtenu dans l'étape ci-dessus; dans 100 $cm^3$ d'acide chlorhydrique 5N et chauffe sous agitation 3 heures au reflux. On refroidit, ajoute 150 $cm^3$ d'eau et extrait le mélange avec de l'éther éthylique (2 fois 150 $cm^3$). On lave les extraits organiques réunis avec 4 fois 50 $cm^3$ d'eau, 50 $cm^3$ d'une solution saturée en bicarbonate de sodium, sèche sur sulfate de magnésium, filtre et concentre à sec. Le résidu de l'évaporation est chromatographié sur une colonne de 3,8 cm de diamètre, contenant 160 g de gel de silice (50 à 200 μ). On élue par 2,7 litres d'un mélange d'oxyde de diisopropyle-méthanol (98-2 en volumes), en recueillant des fractions de 150 $cm^3$. On concentre à sec les fractions comprises entre 1,2 et 2,7 litres et on obtient 7,5 g (90%) de (bis(méthoxy-4 phényl)-4,5 oxazolyl-2]-7 heptanone-2 sous la forme d'une huile jaune pâle.

Spectre de RMN du proton (200 MHz, $CDCl_3$, δ en ppm et J en Hz):
2,14 (s, $-OC-CH_3$);
2,47 (t, J=7, $-CH_2-CO-$);
2,83 (t, J=7,5, $-N=C-CH_2-$);
3,82 (s, deux $-OCH_3$).

On dissout l'heptanone obtenue ci-dessus dans 90 $cm^3$ d'éthanol contenant 3,9 g de chlorhydrate d'hydroxylamine en solution dans 8 $cm^3$ d'eau. Le mélange est additionné de 8,8 g de pyridine puis chauffé 6 heures à reflux. Après refroidissement, on concentre à sec et ajoute 50 $cm^3$ d'eau et 200 $cm^3$ d'oxyde de diéthyle. On décante la phase éthérée et lave celle-ci par 5 fois 40 $cm^3$ d'eau, 30 $cm^3$ d'une solution saturée en chorure de sodium, sèche sur sulfate de magnésium, filtre et concentre sous pression réduite (5,2 kPa). On obtient 9g de [bis(méthoxy-4 phényl)-4,5 oxazolyl-2]-7 hydroxyimino-2 heptane, sous la forme d'une huile visqueuse, directement mise en oeuvre dans l'étape ultérieure.

Spectre de RMN du proton, mélange des oximes isomères (E) et (Z) (200 MHz, $CDCl_3$, δ en ppm et J en Hz) :
1,86 et 1,88 (2s, $-C-CH_3$ des deux isomères);
2,83 (t, J=7,5, $-N=C-CH_2-$);
7,8 à 8,5 et 8,62 (2 mf, =N-OH des deux isomères).

## EXEMPLE 8

Selon l'exemple 5, on chauffe 1,45 g d'oxalate acide de N-{[bis(chloro-4 phényl)-4,5 oxazolyl-2]-6 hexyl} hydroxylamine dans 55 $cm^3$ d'acide acétique contenant 1,5 g d'anhydride acétique. En opèrant comme décrit à l'exemple 5 on obtient une huile jaunâtre. On chromatographie celle-ci sur une colonne de 2 cm de diamètre contenant 30 g de gel de silice (50 à 200 μ). On élue avec 0,4 litre d'un mélange à parties égales d'oxyde de diisopropyle-acétate d'éthyle en recueillant des fractions de 25 $cm^3$. Les fractions comprises entre 0,15 et 0,4 litre sont concentrées à sec sous pression réduite. On dissout le résidu obtenu (1,3 g) dans 20 $cm^3$ de méthanol contenant 5 $cm^3$ d'ammoniaque à 33%. Après agitation et traitement, on chromatographie le résidu obtenu sur une colonne de 2 cm de diamètre contenant 15 g de gel de silice (50 à 200 μ). On élue par 0,3 litre d'acétate d'éthyle, en recueillant des fractions de 20 $cm^3$. On concentre à sec sous pression réduite (0,2 kPa) les fractions comprises entre 0,1 et 0,3 litre et cristallise le résidu huileux dans 20 $cm^3$ d'hexane. On obtient 0,8 g (67%) d'acide N-{[bis(chloro-4 phényl)-4,5 oxazolyl-2]-6 hexyl} acétohydroxamique, sous la forme de cristaux blancs fondant à 78°C.

## EXEMPLE 9

Selon l'exemple 7, on agite à 110°C pendant 3 heures, 2,5 g d'oxalate acide de N-{[bis(chloro-4 phényl)-4,5 oxazolyl-2]-5 pentyl} hydroxylamine dans 50 $cm^3$ d'acide acétique contenant 3,2 g d'anhydride acétique. Après traitement, on obtient un résidu huileux qui est chromatographié sur une colonne de 2 cm de diamètre, contenant 20 g de gel de silice (50 à 200 μ). On élue, en recueillant des fractions de 20 $cm^3$, par 0,3 litre d'un mélange d'oxyde de diisopropyle-acétate d'éthyle (50-50 en volume). On concentre à sec les fractions comprises entre 0,1 et 0,3 litre. On dissout le résidu obtenu (2 g) dans 50 $cm^3$ de méthanol contenant 10 $cm^3$ d'ammoniaque à 33%. On agite le mélange pendant 2 heures à 20°C. Après traitement, comme dans l'exemple 7, le résidu huileux est chromatographié sur une colonne de 2 cm de diamètre, contenant 20 g de gel de silice (50 à 200 μ). On élue par 0,2 litre d'acétate d'éthyle, en recueillant des fractions de 20 $cm^3$. On concentre à sec sous pression réduite (0,2 kPa) les fractions comprises entre 0,1 et 0,2 litre. On obtient 1,5 g (82%) d'acide N-{[bis(chloro-4 phényl)-4,5 oxazolyl-2]-5 pentyl} acétohydroxamique sous la forme d'une huile jaune clair.

Spectre de RMN du proton (250 MHz, $CDCl_3$, δ en ppm, J en Hz à 340°K) :
2,04 (s, $>N-CO-CH_3$);
2,91 (t, J=7,5, $-N=C-CH_2-$);
3,7 (t, J=7, $>N-CH_2-$).

On peut préparer l'oxalate acide de N-{[bis(chloro-4 phényl)-4,5 oxazolyl-2]-5 pentyl} hydroxylamine de la manière suivante:

On chauffe sous agitation pendant 16 heures à 120°C, 10 g de {[bis(chloro-4 phényl)-4,5 oxazolyl-2]-5 pentyl}-2 dihydro-2,5 méthyl-3 oxo-5 isoxazolecarboxylate-4 d'éthyle en solution dans 50 $cm^3$ d'acide acétique, avec 100 $cm^3$ d'acide chlorhydrique 6N. Le mélange est ramené à 60°C puis concentré à sec sous pression réduite (5,2 kPa). Le résidu est dissous dans 0,1 litre d'eau, et la solution lavée avec 0,1 litre d'oxyde de diéthyle. La solution aqueuse décantée est additionnée de 10 $cm^3$ de soude 5N et extraite avec 2 fois 50 $cm^3$ d'oxyde de diéthyle. Les extraits éthérés réunis sont lavés avec 50 $cm^3$ d'une solution saturée de chorure de sodium,

séchés sur sulfate de magnésium, filtrés et le solvant évaporé. Le résidu, en solution dans 100 $cm^3$ d'oxyde de diéthyle, est salifié par addition d'une solution de 2 g d'acide oxalique dans 20 $cm^3$ d'acétate d'éthyle. Le sel obtenu est essoré , et recristallisé dans 100 $cm^3$ d'éthanol. On obtient 5,5 g (60%) d'oxalate acide de N-{[bis(chloro-4 phényl)-4,5 oxazolyl-2]-5 pentyl} hydroxylamine sous la forme de cristaux blancs fondant à 145°C.

Selon l'exemple 1, on peut préparer le {[bis(chloro-4 phényl)-4,5 oxazolyl-2]-5 pentyl}-2 dihydro-2,5 méthyl-3 oxo-5 isoxazolecarboxylate-4 d'éthyle. On chauffe sous agitation, 8 g de sel de sodium du méthyl-3 hydroxy-5 isoxazolecarboxylate-4 d'éthyle en suspension dans 100 $cm^3$ de diméthylformamide avec 15 g de bis(choro-4 phényl)-4,5 (bromo-5 pentyl)-2 oxazole et 1 g d'iodure de potassium. On recristallise le produit issu de la réaction dans 100 $cm^3$ d'acétate d'isopropyle et on obtient 10 g (55%) d'une poudre blanche fondant à 128°C.

On peut préparer le bis(choro-4 phényl)-4,5 (bromo-5 pentyl)-2 oxazole de la manière suivante:

On chauffe sous agitation pendant 3 heures à 110°C, 17 g de bis(chloro-4 phényl)-1,2 (bromo-6 hexanamido)-2 éthanone dans 100 $cm^3$ de toluène, et 14,2 g de tribromure de phosphore. A température ambiante, on additionne 100 $cm^3$ d'oxyde de diéthyle et 100 $cm^3$ d'une solution de soude 2N. On agite 0,5 heure et décante la phase organique. On lave celle-ci par 3 fois 50 $cm^3$ d'eau, 40 $cm^3$ d'une solution saturée en chlorure de sodium, sèche sur sulfate de magnésium, filtre et concentre sous pression réduite (5,2 kPa). Le résidu huileux obtenu cristallise dans 100 $cm^3$ d'oxyde de diisopropyle. On obtient 15 g (92%) de critaux blancs fondant à 92°C.

On peut préparer la bis (chloro-4 phényl)-1,2 (bromo-6 hexanamido)-2 éthanone selon l'exemple 1:

A partir de 15 g de chlorhydrate d'amino-2 bis(chloro-4 phényl)-1,2 éthanone dans 100 $cm^3$ de dichlorométhane contenant 11,8 g de chlorure de l'acide bromo-6 hexanoïque , on ajoute 10 g de pyridine dans 10 $cm^3$ de dichlorométhane. On recristallise le résidu solide dans 150 $cm^3$ d'oxyde de diisopropyle. On isole 17,5 g (80%) de cristaux blancs fondant à 105°C.

## EXEMPLE 10

Selon l'exemple 7, on agite 2,9 g de N-acétoxy N-{[bis(chloro-4 phényl)-4,5 oxazolyl-2]-7 heptyl-2} acétamide, dans 60 $cm^3$ de méthanol contenant 10 $cm^3$ d'ammoniaque à 33%. On extrait le produit de la réaction comme dans l'exemple 7 et cristallise celui-ci dans 100 $cm^3$ d'oxyde de diisopropyle. On obtient 2,2 g (83%) d'acide N-{[bis(chloro-4 phényl)-4,5 oxazolyl-2]-7 heptyl-2} acétohydroxamique (R,S) sous la forme de cristaux fondant à 100°C.

On peut préparer le N-acétoxy N-{[bis(chloro-4 phényl)-4,5 oxazolyl-2]-7 heptyl-2} acétamide comme suit:

A partir de 3 g de N-{[bis(chloro-4 phényl)-4,5 oxazolyl-2]-7 heptyl-2} hydroxylamine (R,S), préparée selon l'exemple 4, dans 30 $cm^3$ d'acide acétique contenant 3 g d'anhydride acétique, on obtient après extraction un résidu huileux. Celui-ci est chromatographié sur une colonne de 2 cm de diamètre, contenant 40 g de gel de silice (50 à 200 μ). On élue, en recueillant des fractions de 100 $cm^3$, par 0,4 litre d'oxyde de diisopropyle puis par 0,8 litre d'un mélange d'oxyde de diisopropyle-méthanol (98-2 en volume). On concentre à sec les fractions réunies du dernier mélange éluant. On obtient 3 g (83%) de N-acétoxy N-{[bis(chloro-4 phényl)-4,5 oxazolyl-2]-7 heptyl-2} acétamide sous la forme d'une huile incolore utilisée dans la réaction ultérieure.

Spectre de RMN du proton (250 MHz, $CDCl_3$, δ en ppm, J en Hz à 333°K) :
1,16 (d, J=7, $-CH_3$);
2,01 et 2,21 (2s, $CH_3$-CO-N-$OCOCH_3$);
2,83 (t, J=7,5, -N=C-$CH_2$-);
4,55 (mt, >N-CH<).

## EXEMPLE 11

A une solution de 1,4 g de N-acétoxy N-{[(chloro-4 phényl)-4 (méthoxy-4 phényl)-5 oxazolyl-2]-6 hexyl} acétamide dans 40 $cm^3$ de méthanol on ajoute 20 $cm^3$ d'ammoniaque à 33%. On agite le mélange 2 heures à 20°C. Après évaporation sous pression réduite, on ajoute au résidu 10 $cm^3$ d'eau, extrait avec de l'acétate d'ethyle (2 fois 40 $cm^3$). On lave les extraits organiques réunis avec 3 fois 30 $cm^3$ d'eau et 20 $cm^3$ d'une solution saturée en chlorure de sodium. On sèche sur sulfate de magnésium, filtre et concentre à sec. Le résidu huileux obtenu cristallise dans 30 $cm^3$ d'oxyde de diisopropyle. On obtient 1 g (78%) d'acide N-{[(chloro-4 phényl)-4 (méthoxy-4 phényl)-5 oxazolyl-2]-6 hexyl} acétohydroxamique, sous la forme de cristaux blancs fondant à 74°C.

On peut préparer le N-acétoxy N-{[(chloro-4 phényl)-4 (méthoxy-4 phényl)-5 oxazolyl-2]-6 hexyl} acétamide de la manière suivante:

On ajoute à une solution de 3,5 g de [(chloro-4 phényl)-4 (méthoxy-4 phényl)-5 oxazolyl-2]-6 hexanal

dans 50 $cm^3$ d'éthanol une solution de 1,5 g de chlorhydrate d'hydroxylamine dans 5 $cm^3$ d'eau et 4 g de pyridine. On chauffe sous agitation 6 heures au reflux et concentre à sec sous pression réduite. On ajoute au résidu 40 $cm^3$ d'eau, extrait avec de l'éther éthylique (2 fois 50 $cm^3$) , lave les extraits organiques réunis avec 3 fois 30 $cm^3$ d'eau, sèche sur sulfate de magnésium, filtre et concentre à sec. On chromatographie le produit de la réaction sur une colonne de 2,8 cm de diamètre, contenant 40 g de gel de silice (50 à 200 μ). On élue, en recueillant des fractions de 20 $cm^3$, par 0,4 litre d'oxyde de diisopropyle. On concentre à sec les fractions comprises entre 0,1 et 0,4 litre. On obtient 3,3 g d'une huile jaunâtre. On dissout celle-ci dans 50 $cm^3$ d'acide acétique et additionne 1,5 g de cyanoborohydrure de sodium. On agite le mélange 2 heures à 10°C. On traite celui ci à 0°C par addition de 100 $cm^3$ de soude 10N et extrait par 2 fois 100 $cm^3$ d'oxyde de diéthyle. Les extraits organiques réunis sont lavés par 4 fois 40 $cm^3$ d'eau, séchés sur sulfate de magnésium, filtrés et concentrés sous vide. On obtient 2,2 g d'une huile incolore que l'on dissout dans 20 $cm^3$ d'acide acétique avec 4,3 g d'anhydride acétique. La solution est chauffée 2 heures à 100°C. On concentre à sec sous pression réduite à 60°C. On ajoute au résidu 50 $cm^3$ d'eau et 100 $cm^3$ d'oxyde de diéthyle, agite 1 heure et décante. On lave les extraits organiques par 4 fois 30 $cm^3$ d'eau, sèche sur sulfate de magnésium, filtre et concentre à sec. On chromatographie l'huile obtenue sur une colonne de 2 cm de diamètre, contenant 30 g de gel de silice (50 à 200 μ). On élue par 0,4 litre d'un mélange à parties égales (vol/vol) d'oxyde de diisopropyle et d'acétate d'éthyle, en recueillant des fractions de 20 $cm^3$. On concentre à sec les fractions comprises entre 0,2 et 0,4 litre. On obtient une huile qui cristallise dans 30 $cm^3$ d'oxyde de diisopropyle. On obtient 2,2 g (82%) de N-acétoxy N-{[(chloro-4 phényl)-4 (méthoxy-4 phényl)-5 oxazolyl-2]-6 hexyl} acétamide, sous la forme de cristaux blancs fondant à 80°C.

On peut préparer le [(chloro-4 phényl)-4 (méthoxy-4 phényl)-5 oxazolyl-2]-6 hexanal de la manière suivante:

On agite 4 heures à 20°C, 4,3 g de [(chloro-4 phényl)-4 (méthoxy-4 phényl)-5 oxazolyl-2]-6 hexanol dans 70 $cm^3$ de dichlorométhane et 3,6 g de chlorochromate de pyridinium. On filtre le précipité sur clarcel et concentre sous vide le filtrat. On chromatographie l'huile obtenue sur une colonne de 2 cm de diamètre, contenant 50 g de gel de silice (50 à 200 μ). On élue, en recueillant des fractions de 20 $cm^3$, par 0,4 litre d'oxyde de diisopropyle . On concentre à sec les fractions comprises entre 0,1 et 0,4 litre. On obtient une huile qui cristallise dans 30 $cm^3$ d'hexane. On obtient 3,5 g (81%) de cristaux blancs fondant à 60°C.

On peut préparer le [(chloro-4 phényl)-4 (méthoxy-4 phényl)-5 oxazolyl-2]-6 hexanol de la manière suivante:

Selon l'exemple 1, à une suspension agitée et refroidie à 5°C de 6, 2 g de chlorhydrate d'amino-2 (chloro-4 phényl)-2 (méthoxy-4 phényl)-1 éthanone [G. DREFAHL, G. HEUBLEIN, K. FRITZSCHE et R. SIEMANN, J. Prakt. Chem., 32, p 307 à 310, (1966)] dans 50 $cm^3$ de dichlorométhane contenant 4,9 g de chlorure de bromo-7 heptanoyle, on ajoute 4 g de pyridine dans 5 $cm^3$ de dichlorométhane. On isole, comme décrit à l'exemple 1, 8 g d'une huile épaisse jaunâtre. On traite la solution de celle-ci dans 50 $cm^3$ de toluène par 3 g d'oxychlorure de phosphore. On purifie le produit issu de la réaction par chromatographie sur une colonne de 2,8 cm de diamètre, contenant 150 g de gel de silice (50 à 200 μ). On élue par 0,5 litre d'un mélange à parties égales (vol/vol) d'oxyde de diisopropyle et d'hexane, en recueillant des fractions de 50 $cm^3$. On concentre à sec les fractions comprises entre 0,2 et 0,5 litre. On obtient 7 g d'une huile jaune clair. On dissout celle-ci dans 100 $cm^3$ de méthyléthylcétone, ajoute 3,1 g d'acétate de potassium, 0,05 g de bromure de tétraéthylammonium et chauffe le mélange 20 heures à 100°C. On refroidit, essore le résidu minéral, et concentre sous vide. On chromatographie le résidu obtenu sur une colonne de 2,5 cm de diamètre, contenant 50 g de gel de silice (50 à 200 μ). On élue, en recueillant des fractions de 50 $cm^3$, par 0,7 litre d'un mélange à parties égales (vol/vol) d'oxyde de diisopropyle et d'hexane. On concentre à sec les fractions comprises entre 0,2 et 0,7 litre et obtient 6 g d'une huile jaune clair. On dissout celle-ci dans 50 $cm^3$ de méthanol, ajoute 25 $cm^3$ d'ammoniaque à 33%, et agite la solution 10 heures au reflux. On concentre sous pression réduite (5,2 kPa). On purifie par chromatographie le résidu obtenu sur une colonne de 2,5 cm de diamètre, contenant 50 g de gel de silice (50 à 200 μ). On élue, par 0,2 litre d'oxyde de diisopropyle, puis par 0,4 litres d'acétate d'éthyle, en recueillant des fractions de 50 $cm^3$. On concentre à sec les éluats de ce dernier solvant, et cristallise le résidu huileux dans 30 $cm^3$ d'hexane. On obtient 4,3 g (79%) de [(chloro-4 phényl)-4 (méthoxy-4 phényl)-5 oxazolyl-2]-6 hexanol sous forme de cristaux blancs fondant à 76°C.

EXEMPLE 12 RP 72665

Selon les conditions définies dans l'exemple 5, on traite 4 g d'oxalate acide de N-[(diphényl-4,5 oxazolyl-2)-6 hexyl] hydroxylamine, avec 50 $cm^3$ d'acide acétique et 5,4 g d'anhydride acétique. Le produit, après traitement, est chromatographié sur une colonne de 2 cm de diamètre contenant 25 g de gel de silice (50 à 200 μ). On élue avec 0,24 litre d'un mélange d'oxyde de diisopropyle-acétate d'éthyle (65-35 en volume) en recueillant des fractions de 20 $cm^3$. Les fractions comprises entre 60 et 240 $cm^3$ sont concentrées à sec. On dissout le résidu (3,5 g) dans 50 $cm^3$ de méthanol contenant 10 $cm^3$ d'ammoniaque à 33%, puis on agite le mélange pendant 2 heures à 20°C. Le produit, après traitement, est chromatographié sur une colonne de 2 cm de diamètre contenant 20 g de gel de silice (50 à 200 μ). On élue, en recueillant des fractions de 20 $cm^3$, par 0,24 litre d'acétate d'éthyle. On concentre à sec sous pression réduite (0,2 kPa) les fractions comprises entre 0,1 et 0,24 litre. On obtient ainsi 1,9 g (70%) d'acide N-[(diphényl-4,5 oxazolyl-2)-6 hexyl] acétohydroxamique, sous la forme d'une huile jaunâtre.

Spectre de RMN du proton (300 MHz, CDCl3, δ en ppm,et J en Hz) :
2,1 (s, $-COCH_3$);
2,85 (mt, $-N=C-CH_2-$);
3,62 (mt, $N-CH_2-$);
8,6 et 9,4 (2mf, >N-OH des deux rotamères).

L'oxalate acide de N-[(diphényl-4,5 oxazolyl-2)-6 hexyl] hydroxylamine peut être préparé de la manière suivante:

Selon les conditions (temps et température) définies dans l'exemple 1, on traite 38,1 g de [(diphényl-4,5 oxazolyl-2)-6 hexyl]-2 dihydro-2,5 méthyl-3 oxo-5 isoxazolecarboxylate-4 d'éthyle en solution dans 80 $cm^3$ d'acide acétique pur avec 160 $cm^3$ d'acide chlorhydrique 6N. On extrait le produit de la réaction comme dans l'exemple cité. Le résidu, en solution dans 100 $cm^3$ de dichlorométhane, est salifié par addition de 6,4 g d'acide oxalique anhydre. Le solide résultant est essoré et séché. On obtient 21,8 g (63%) d'oxalate acide de N-[(diphényl-4,5 oxazolyl-2)-6 hexyl] hydroxylamine sous la forme d'une poudre blanche fondant à 153°C.

On peut préparer le [(diphényl-4,5 oxazolyl-2)-6 hexyl]-2 dihydro-2,5 méthyl-3 oxo-5 isoxazolecarboxylate-4 d'éthyle selon l'exemple 1:

On chauffe, pendant 20 heures à 105°C, 17,5 g de sel de sodium du méthyl-3 hydroxy-5 isoxazolecarboxylate-4 d'éthyle dans 70 $cm^3$ de diméthylformamide avec 33 g de diphényl-4,5 (bromo-6 hexyl)-2 oxazole et 0,8 g d'iodure de potassium. Après traitement, on chromatographie le produit obtenu sur une colonne de 4,4 cm de diamètre, contenant 300 g de gel de silice (50 à 200 μ). On élue, en recueillant des fractions de 250 $cm^3$, par 1 litre d'oxyde de diisopropyle puis par 1,5 litre d'un mélange à parties égales (vol/vol) d'oxyde de diisopropyle et d'acétate d'éthyle. On concentre à sec les fractions du dernier mélange.On obtient 38,1 g (93%) de [(diphényl-4,5 oxazolyl-2)-6 hexyl]-2 dihydro-2,5 méthyl-3 oxo-5 isoxazolecarboxylate-4 d'éthyle sous la forme d'une huile jaunâtre directement utilisée dans la réaction ultérieure.

Spectre de RMN du proton ( 250 MHz, $CDCl_3$, δ en ppm et J en Hz) :
1,33 (t, J=7, $-CH_2-CH_3$);
2,49 (s, $-CH_3$);
2,83 (t, J=7,5, $-N=C-CH_2-$);
3,84 (t, J=7, $>N-CH_2-$);
4,27 (q, J=7, $-COOCH_2CH_3$).

On peut préparer le diphényl-4,5 (bromo-6 hexyl)-2 oxazole selon l'exemple 4.

On chauffe sous agitation pendant 4 heures à 110°C, 42,4 g de diphényl-1,2 (bromo-7 heptanamido)-2 éthanone dans 100 $cm^3$ de toluène, avec 34,2 g de tribromure de phosphore. Après traitement, on chromatographie le produit de la réaction sur une colonne de 4,4 cm de diamètre, contenant 300 g de gel de silice (50 à 200 μ). On élue, en recueillant des fractions de 200 $cm^3$, par 2 litres d'oxyde de diisopropyle. On concentre à sec sous pression réduite (5,2 kPa) les fractions comprises entre 0,4 et 2 litres. On obtient 33 g (81%) de diphényl-4,5 (bromo-6 hexyl)-2 oxazole sous la forme d'une huile jaunâtre directement utilisée dans la réaction ultérieure.

Spectre de RMN du proton (300 MHz, $CDCl_3$, δ en ppm et J en Hz) :
2,86 (t, J=7, -N=C-CH2-);
3,4 (t, J=7, $-CH_2-Br$).

On peut préparer le diphényl-1,2 (bromo-7 heptanamido)-2 éthanone selon l'exemple 1.

A partir de 49,4 g de chlorhydrate d'amino-2 diphényl-1,2 éthanone dans 240 cm3 de dichlorométhane contenant 47,8 g de chlorure de l'acide bromo-7 heptanoïque, on ajoute 39,7 g de pyridine dans 50 $cm^3$ de dichlorométhane. Le produit, après traitement, est recristallisé dans 70 $cm^3$ d'acétate d'isopropyle. On obtient 42,4 g (52%) de cristaux blancs fondant à 93°C.

## EXEMPLE 13

Selon les conditions (temps et température) définies dans l'exemple 6, on traite 3 g d'oxalate acide de N-[(diphényl-4,5 oxazolyl-2)-6 hexyl] hydroxylamine en solution dans 30 $cm^3$ de tétrahydrofuranne et 30 $cm^3$ d'eau contenant 1,85 g de carbonate de potassium par 0,83 g de chlorure d'isobutyryle dilué dans 2 $cm^3$ de tétrahy-

drofuranne. On chromatographie le produit de la réaction, isolé selon l'exemple 8 cité, sur une colonne de 1,6 cm de diamètre, contenant 20 g de gel de silice (50 à 200 μ). On élue par 0,2 litre de dichlorométhane puis par 0,7 litre d'un mélange de dichlorométhane-méthanol (90-10 en volume), en recueillant des fractions de 50 $cm^3$. Les fractions du dernier mélange éluant sont concentrées à sec sous pression réduite (0,2 kPa). On obtient 1,9 g (66%) de N-[(diphényl-4,5 oxazolyl-2)-6 hexyl] N-hydroxyisobutyramide, sous la forme d'une huile jaunâtre.

Spectre de RMN du proton (200 MHz, $CDCl_3$ plus quelques gouttes de $CD_3OD$ d4, δ en ppm et J en Hz) :
1 (d, J=6,5, $-CH-(CH_3)_2$);
2,75 (t, J=7,5, $-N=C-CH_2-$);
3,06 (mt, >N-CO-CH<);
3,5 (t, J=7, $>N-CH_2-$).

EXEMPLE 14

Selon l'exemple 9, on agite à 110°C pendant 3 heures, 3 g d'oxalate acide de N-[(diphényl-4,5 oxazolyl-2)-5 pentyl] hydroxylamine, dans 50 $cm^3$ d'acide acétique contenant 3,8 g d'anhydride acétique. Après traitement, on chromatographie l'huile obtenue sur une colonne de 2 cm de diamètre, contenant 20 g de gel de silice (50 à 200 μ). On élue ,par 0,85 litre d'acétate d'éthyle, en recueillant des fractions de 50 $cm^3$. On concentre à sec les fractions comprises entre 0,15 et 0,85 litre. On dissout le résidu (2,5 g) dans 50 $cm^3$ de méthanol contenant 10 $cm^3$ d'ammoniaque à 33%., puis agite le mélange pendant 2 heures à 20°C. Après traitement, selon l'exemple 9, le résidu huileux est chromatographié sur une colonne de 2 cm de diamètre, contenant 20 g de gel de silice (50 à 200 μ). On élue, en recueillant des fractions de 50 $cm^3$, par 0,6 litre d'acétate d'éthyle. On concentre à sec sous pression réduite (0,2 kPa), les fractions comprises entre 0,1 et 0,6 litre. On obtient 1,4 g (63%) d'acide N-[(diphényl-4,5 oxazolyl-2)-5 pentyl] acétohydroxamique sous la forme d'une huile incolore.

Spectre de RMN du proton (200MHz, DMSO d6 plus quelques gouttes de $CD_3COOD$ d4, δ en ppm et J en Hz) :
1,98 (s, $>N-COCH_3$);
2,84 (t, J=7,5, $-N=C-CH_2-$);
3,52 (t, J=7, $>N-CH_2-$).

On peut préparer l'oxalate acide de N-[(diphényl-4,5 oxazolyl-2)-5 pentyl] hydroxylamine, selon les conditions définies dans l'exemple 1 :

On traite 18,6 g de [(diphényl-4,5 oxazolyl-2)-5 pentyl]-2 dihydro-2,5 méthyl-3 oxo-5 isoxazolecarboxylate-4 d'éthyle en solution dans 40 $cm^3$ d'acide acétique pur avec 80 $cm^3$ d'acide chlorhydrique 6N. On extrait le produit de la réaction comme dans l'exemple 1. Le résidu, en solution dans 150 $cm^3$ de dichlorométhane, est salifié par addition de 3,1 g d'acide oxalique anhydre. Le solide résultant est essoré et séché. On obtient 10,65 g (64%) d'oxalate acide de N-[(diphényl-4,5 oxazolyl-2)-5 pentyl] hydroxylamine sous la forme d'une poudre blanche fondant à 139°C.

On peut préparer le [(diphényl-4,5 oxazolyl-2)-5 pentyl]-2 dihydro-2,5 méthyl-3 oxo-5 isoxazolecarboxylate-4 d'éthyle selon l'exemple 1 :

On chauffe, pendant 20 heures à 105°C, 13,6 g de sel de sodium du méthyl-3 hydroxy-5 isoxazolecarboxylate-4 d'éthyle dans 70 $cm^3$ de diméthylformamide avec 25,2 g de diphényl-4,5 (bromo-5 pentyl)-2 oxazole et 0,7 g d'iodure de potassium. Après traitement, on chromatographie le produit obtenu sur une colonne de 4,4cm de diamètre, contenant 260 g de gel de silice (50 à 200μ). On élue, en recueillant des fractions de 250 $cm^3$, par 5 litres d'un mélange d'oxyde de diisopropyle-acétate d'éthyle (80-20 en volume), puis par 4,5 litres d'un mélange à parties égales (vol/vol) d'oxyde de diisopropyle et d'acétate d'éthyle. On concentre à sec les fractions du dernier mélange. On obtient 18,6 g (59%) de [(diphényl-4,5 oxazolyl-2)-5 pentyl]-2 dihydro-2,5 méthyl-3 oxo-5 isoxazolecarboxylate-4 d'éthyle sous la forme d'une huile jaunâtre directement utilisée dans la réaction ultérieure.

Spectre de RMN du proton (300 MHz, $CDCl_3$, δ en ppm, J en Hz)
1,35 (t, J=7, $-CH_2-CH_3$);
2,55 (s, $-CH_3$);
2,87 (t, J=7, $-N=C-CH_2-$);
3,89 (:, J=7, $-CH_2-N$);
4,31 (q, J-7, $-COOCH_2-CH_3$).

On peut préparer le diphényl-4,5 (bromo-5 pentyl)-2 oxazole selon l'exemple 1.

On chauffe sous agitation pendant 4 heures à 110°C, 50,5 g de diphényl-1,2 (bromo-6 hexanamido)-2 éthanone dans 130 $cm^3$ de toluène, avec 42,2 g de tribromure de phosphore. Après traitement, on chromatographie le produit de la réaction sur une colonne de 4,4 cm de diamètre, contenant 200 g de gel de silice (50 à 200μ).

On élue, en recueillant des fractions de 300 $cm^3$, par 3 litres d'hexane puis par 4,5 litres d'un mélange d'oxyde de diisopropyle-hexane (10-90 en volume). On concentre à sec sous pression réduite (5,2 kPa) les fractions réunies du dernier mélange éluant. On obtient 25,3 g (52%) de diphényl-4,5 (bromo-5 pentyl)-2 oxazole sous la forme d'une huile jaunâtre directement utilisée dans la réaction ultérieure.

Spectre de RMN du proton (300MHz, $CDCl_3$, $\delta$ en ppm, J en Hz)
2,9 (t, J=7,5, $-N=C-CH_2-$);
3,45 (t, J7, $-CH_2-Br$).

On peut préparer le diphényl-1,2 (bromo-6 hexanamido)-2 éthanone selon l'exemple 1.

A partir de 12,9 g de chlorhydrate d'amino-2 diphényl-1,2 éthanone dans 63 $cm^3$ de dichlorométhane contenant 11,7 g de chlorure de l'acide bromo-6 hexanoïque, on ajoute 10,3 g de pyridine dans 13 $cm^3$ de dichlorométhane. Le produit, après traitement, est recristallisé dans 100 $cm^3$d'oxyde de diisopropyle. On obtient 15,1 g (74%) de cristaux blancs fondant à 92°C.

La présente invention concerne également les compositions pharmaceutiques constituées par un produit de formule générale (I), ou un sel, éventuellement en association avec tout autre produit compatible, pouvant être inerte ou physiologiquement actif. Les compositions selon l'invention peuvent être utilisées par voie parentérale, orale, rectale ou topique.

Comme compositions solides pour administration orale, peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive, et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui seront dissoutes au moment de l'emploi dans de l'eau stérile ou tout autre milieu stérile injectable.

Les compositions pour administration rectale sont les suppositoires ou les capsules rectales qui peuvent contenir, outre le produit actif, des excipients tels que le beurre de cacao ou la suppo-cire.

Les compositions pour administration topique peuvent être par exemple des crèmes, des pommades, ou des lotions.

En thérapeutique humaine, les produits selon l'invention peuvent être particulièrement utiles dans le traitement des maladies d'origine inflammatoire. Ils peuvent donc s'avérer très utiles en pathologie ostéoarticulaire dans le traitement de l'arthrite, de la polyarthrite rhumatoïde, de la spondylarthrite , de la goutte, de l'arthrose, de la chondrocalcinose, ainsi que dans d'autres pathologies inflammatoires touchant les poumons, les voies digestives (colite ulcéreuse, inflammation hépatique, cirrhose, maladies du colon, maladie de Crohn), la peau (psoriasis, herpès, acné, érythème, eczéma, dermites), les yeux, les voies nasales, la cavité buccale et les dents. Ils peuvent également être utilisés dans les traitements des allergies nasales et bronchiques (asthme). Les produits selon l'invention peuvent aussi être utiles dans les traitements des inflammations liées à la mise en place d'implants, en améliorant leur compatibilité avec le tissu environnant. Ils peuvent également jouer un rôle dans l'immunorégulation (maladies auto-immunes), l'ischémie et la reperfusion (cardiaque notamment).

Ces produits exercent aussi un effet bénéfique dans le traitement de l'hyperthermie et de la douleur.

Les doses dépendent de l'effet recherché et de la durée du traitement. Pour un adulte elles sont généralement comprises entre 500 mg et 2 g par jour par voie orale. D'une manière générale, le médecin déterminera la dose qu'il estime la plus appropriée en fonction de l'âge, du poids et de tous les autres facteurs propres au sujet à traiter.

L'exemple suivant illustre une composition selon l'invention.

EXEMPLE

On prépare selon la technique habituelle, des comprimés de produit actif ayant la composition suivante :

| | |
|---|---|
| - N-{[Bis(méthoxy-4 phényl)-4,5 oxazolyl-2]-6 hexyl} N-hydroxyurée ........ ............................... | 100 mg |
| - amidon ............................................. | 332 mg |
| - silice ............................................. | 120 mg |
| - stéarate de magnésium .............................. | 12 mg |

## Revendications

**1 -** Nouveau dérivé de l'oxazole de formule générale :

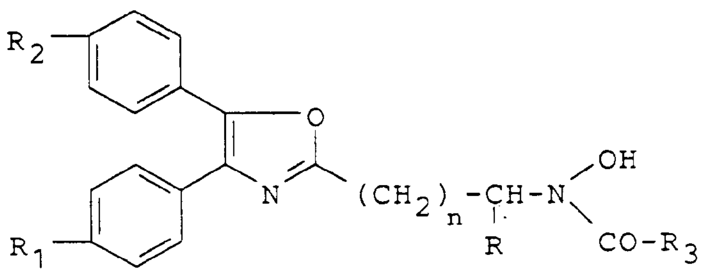

dans laquelle :
R est un atome d'hydrogène ou un radical alcoyle contenant 1 ou 2 atomes de carbone,
$R_1$ et $R_2$ sont identiques ou différents et représentent des atomes d'hydrogène ou d'halogène, ou des radicaux alcoyloxy dont la partie alcoyle contient 1 à 4 atomes de carbone en chaîne droite ou ramifiée,
$R_3$ représente un radical amino ou alcoyle contenant 1 à 4 atomes de carbone en chaîne droite ou ramifiée,
n égale 3 à 6,
ainsi que leurs sels et, lorsqu'ils existent, leurs isomères et leurs mélanges.

**2 -** Procédé de préparation d'un dérivé de l'oxazole selon la revendication 1, caractérisé en ce que l'on transforme une hydroxylamine formule générale :

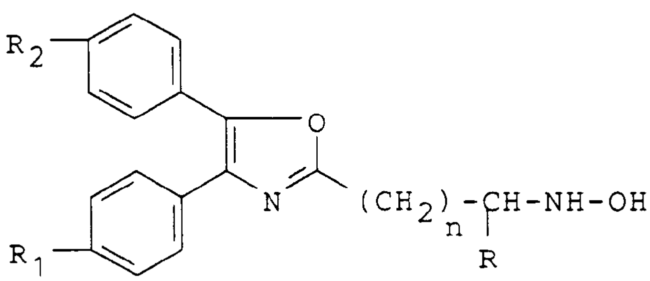

dans laquelle R, $R_1$, $R_2$ et n sont définis comme dans la revendication 1 ou son sel, soit par action d'un cyanate alcalin, soit par acylation au moyen d'un dérivé réactif d'un acide de formule générale :

$$R_3 - COOH$$

dans laquelle $R_3$ est défini comme dans la revendication 1, puis transforme éventuellement le produit obtenu en son sel.

**3 -** Composition pharmaceutique caractérisée en ce qu'elle contient au moins un produit selon la revendication 1, à l'état pur ou sous forme d'association avec un ou plusieurs diluants ou adjuvants compatibles et pharmaceutiquement acceptables.

Office européen des brevets

# RAPPORT DE RECHERCHE EUROPEENNE

Numero de la demande

EP 92 40 1526

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | FR-A-2 081 532 (ISTITUTO FARMACOLOGICO SERONO S.P.A.)<br>* revendications *<br>--- | 1,3 | C07D263/32<br>A61K31/42 |
| A | FR-A-2 085 675 (ISTITUTO FARMACOLOGICO SERONO S.P.A.)<br>* revendications *<br>----- | 1,3 | |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| LA HAYE | 21 AOUT 1992 | HENRY J.C. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P0402)